# EUROPEAN PATENT SPECIFICATION

(11) **EP 2 074 230 B1**
(45) Date of publication and mention of the grant of the patent: **28.11.2012**
(21) Application number: 07818943.8
(22) Date of filing: 11.10.2007
(51) Int. Cl.: C12Q 1/68, G01N 33/53

(54) **COMPOSITIONS AND METHODS FOR TREATING AND DIAGNOSING IRRITABLE BOWEL SYNDROME**
ZUSAMMENSETZUNGEN UND VERFAHREN ZUR BEHANDLUNG UND DIAGNOSE VON REIZKOLON
COMPOSITIONS ET PROCEDES POUR LE TRAITEMENT ET LE DIAGNOSTIC DU SYNDROME DU COLON IRRITABLE

(30) Priority: 11.10.2006 US 850659 P; 14.11.2006 EP 06023597
(43) Date of publication of application: 01.07.2009
(73) Proprietor: Janssen Pharmaceutica NV, 2340 Beerse (BE); Mayo Foundation for Medical Education and Research, Rochester, MN 55905 (US)
(72) Inventor: THIELEMANS, Theodoor Victor Constant, 2340 Beerse (BE); AERSSENS, Jeroen Marcel Maria Roger, 3221 Nieuwrode (BE); TALLOEN, Willem Jan-Paul Edmond, 2600 Antwerpen (BE); THIELEMANS, Leen Albert Leonarda, 2350 Vosselaar (BE); CAMILLERI, Michael L., Rochester, MN 55902 (US)
(74) Representative: Bird, William Edward
(86) International application number: PCT/EP2007/008872
(87) International publication number: WO 2008/043566

(56) References cited:
- WO-A-02/12434
- WO-A-2005/020902
- PARK M-I ET AL: "Genetics and genotypes in irritable bowel syndrome: Implications for diagnosis and treatment" GASTROENTEROLOGY CLINICS OF NORTH AMERICA 2005 UNITED STATES, vol. 34, no. 2 SPEC. ISS., 2005, pages 305-317, XP008078496 ISSN: 0889-8553
- COATES MATTHEW D ET AL: "Molecular defects in mucosal serotonin content and decreased serotonin reuptake transporter in ulcerative colitis and irritable bowel syndrome" GASTROENTEROLOGY, vol. 126, no. 7, June 2004 (2004-06), pages 1657-1664, XP002432328 ISSN: 0016-5085
- OLSEN JORGEN ET AL: "Comparisons of gene expression in non-inflamed colonic mucosa reveal over-representation of genes from IBD susceptibility regions in IBD patients compared with control subjects" GASTROENTEROLOGY, vol. 126, no. 4, Suppl. 2, April 2004 (2004-04), page A355, XP008078502 & DIGESTIVE DISEASE WEEK/105TH ANNUAL MEETING OF THE AMERICAN-GASTROENTEROLOGICAL-ASSOCIATION; NEW ORLEANS, LA, USA; MAY 16 20, 2004 ISSN: 0016-5085
- DATABASE BIOSIS [Online] BIOSCIENCES INFORMATION SERVICE, PHILADELPHIA, PA, US; April 2006 (2006-04), AERSSENS JEROEN ET AL: "Altered expression of TRP, Ca2+ and K+ channels in vagal visceral afferents in a post-infectious model of irritable bowel syndrome (IBS)" XP002433340 Database accession no. PREV200600504217 & GASTROENTEROLOGY, vol. 130, no. 4, Suppl. 2, April 2006 (2006-04), page A441, DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 24, 2006 ISSN: 0016-5085
- SPILLER ROBIN C: "Irritable bowel syndrome" BRITISH MEDICAL BULLETIN, vol. 72, no. 1, 2005, pages 15-29, XP002432329 ISSN: 0007-1420
- NIELSEN O H ET AL: "Intestinal interleukin-8 concentration and gene expression in inflammatory bowel disease" SCANDINAVIAN JOURNAL OF GASTROENTEROLOGY, vol. 32, no. 10, 1997, pages 1028-1034, XP008078412 ISSN: 0036-5521
- GWEE K -A ET AL: "Increased rectal mucosal expression of interleukin 1beta in recently acquired post-infectious irritable bowel syndrome." GUT, vol. 52, no. 4, April 2003 (2003-04), pages 523-526, XP008078461 ISSN: 0017-5749
- DROSSMAN D A ET AL: "AGA technical review on irritable bowel syndrome." GASTROENTEROLOGY, vol. 123, no. 6, December 2002 (2002-12), pages 2108-2131, XP002432330 ISSN: 0016-5085
- DATABASE BIOSIS [Online] BIOSCIENCES INFORMATION SERVICE, PHILADELPHIA, PA, US; April 2006 (2006-04), ANDREWS CHRISTOPHER ET AL: "Serotonin-transporter (SERT) polymorphism genotype and SERT expression in mucosal biopsies of patients with irritable bowel syndrome" XP002433341 Database accession no. PREV200600502261 & GASTROENTEROLOGY, vol. 130, no. 4, Suppl. 2, April 2006 (2006-04), page A24, DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 24, 2006 ISSN: 0016-5085
- BJERRUM J T ET AL: "Genome-wide gene expression analysis of mucosal colonic biopsies and isolated colonocytes suggests a continuous inflammatory state in the lamina propria of patients with quiscient ulcerative colitis", INFLAMMATORY BOWEL DISEASES, WILLAMS AND WILKINS, HAGERSTOWN, MD, US, vol. 16, no. 6, 1 June 2010 (2010-06-01), pages 999-1007, XP002601768, ISSN: 1078-0998, DOI: 10.1002/IBD.21142 [retrieved on 2009-10-15]
- KERCKHOFFS A P M ET AL: "Trypsinogen IV, serotonin transporter transcript levels and serotonin content are increased in small intestine of irritable bowel syndrome patients", NEUROGASTROENTEROLOGY AND MOTILITY 200808 GB LNKD- DOI:10.1111/J.1365-2982.2008.01100.X, vol. 20, no. 8, August 2008 (2008-08), pages 900-907, ISSN: 1350-1925
- VON STEIN P ET AL: "Multigene Analysis Can Discriminate Between Ulcerative Colitis, Crohn's Disease, and Irritable Bowel Syndrome", GASTROENTEROLOGY, ELSEVIER, PHILADELPHIA, PA, vol. 134, no. 7, 1 June 2008 (2008-06-01), pages 1869-1881, XP022694428, ISSN: 0016-5085, DOI: 10.1053/J.GASTRO.2008.02.083 [retrieved on 2008-03-02]
- "Affymetrix Genechip bHuman Genome U133 plus 2.0 Array", GEO, XP002343693,

## Description

The present invention relates generally to diagnosis of disorders associated with chronic visceral hypersensitivity (CVH), and in particular irri bowel syndrome (IBS). In particular this invention relates to polynucleotides encoding polypeptides, wherein said polypeptides are shown to be associated with CVH. These polypeptides and polynucleotides are useful in the diagnosis, treatment and/or prevention of disorders associated with CVH, in particular in the diagnosis, treatment and/or prevention of disorders associated with IBS.

### BACKGROUND OF THE INVENTION

Irritable bowel syndrome (IBS) and inflammatory bowel disease (IBD) represent two conditions characterized by chronically recurring symptoms of abdominal pain, discomfort (urgency and bloating) and alterations in bowel habits. However, these bowel diseases differ significantly in etiology and physiopathology, thus implicating different methods of diagnosis and treatment. Where IBD is characterized by inflammation or ulcerations in the small and/or large intestine, such overt structural changes have not been associated with IBS. IBS is classified as a functional (opposed to an organic) bowel disorder of unknown etiology. A functional disorder refers to a disorder or disease where the primary abnormality is an altered physiological function, rather than an identifiable structural or biochemical cause. Diagnosis of IBS is currently based on a characteristic cluster of symptoms in the absence of detectable structural abnormalities (Drossman DA, Camilleri M, Mayer EA, Whitehead WE. AGA technical review on irritable bowel syndrome. Gastroenterology. 2002; 123:2108-31), including intermittent abdominal pain and discomfort and alterations in bowel habits, such as loose or more frequent bowel movements, diarrhea, and/or constipation that occur in the absence of detectable ongoing organic disease. Because of the lack of specificity of the cardinal symptoms of abdominal pain or abdominal discomfort, the current diagnosis of IBS applies to a heterogeneous group of patients, even after attemps to define subgroups based on predominant bowel habit (diarrhea-predominant, constipation-predominant, alternating diarrhea and constipation, normal). IBS affects approximately 10-20% of the general population. It is the most common disease diagnosed by gastroenterologists and one of the most common disorders seen by primary care physicians.

Current theories to explain the pathophysiology of IBS include alterations in visceral perception, gastrointestinal motility or gut epithelial and immune function. Published evidence demonstrate that IBS is associated with a state of chronic visceral hypersensitivity (CVH) suggesting that processing of visceral sensory information is altered, but the molecular changes underlying the development and maintenance of CVH in IBS are not known. Published evidence supports a role of psychosocial and physical (e.g. enteric infections) stressors as central and peripheral triggers, respectively, which may induce presentation or exacerbation of IBS symptoms. There is for example increasing evidence of a putative role of low-grade chronic inflammation in the pathogenesis of IBS. As a consequence, current medical treatments for IBS primarily target peripheral symptoms rather than the underlying causes, and therapeutic gains from drug treatments are usually modest and the placebo responses are high (Mertz H, Naliboff B, Munakata J, Niazi N, Mayer EA. Altered rectal perception is a biological marker of patients with irritable bowel syndrome. Gastroenterology. 1995; 109: 40-52). Defining the underlying neurological and molecular defects is therefore important to the design of more successful therapeutic strategies. Moreover, there is a need in the art for improved methods for screening, diagnosing and treating IBS and other CVH-related disorders.

In a first effort to try and identify the underlying molecular defects, Pasricha P et al. (PCT publication WO 2005/020902) report the analysis of microarray expression profiles of colon tissue RNA and S1 dorsal root ganglia RNA from a rat model of chronic visceral hypersensitivity upon treatment with CNI-1493.

Park et al. (2005) Gastroent. Clin. 34, 305-317 discloses the genetic implications in the diagnosis and treatment of irritable bowel syndrome (IBS) and reviews a few specific polymorphisms linked to IBS and discloses low mRNA levels of SERT and tryptophan hydroxylase in mucosal biopsies of patients with IBS. This article further discloses that IBS has the difficulty of being a multifactorial disease with many factors influencing and potentially causing the disease. Coates et al. (2004) Gastroenterol. 126 1657-1664 discloses set of marker genes to detect IBS. Olsen et al. (2004) Gastroenterology 126 S2, A355 disclose the expression analysis of genes in samples from IBS patients that are used as control and it mentions data from other expression experiments in colonic inflamed mucosa.
Aerssens et al. (2006) biosciences information service discloses array based studies in a mouse model of IBS. Arrays such as "Affymetrix Genechip Human Genome U133 plus 2.0 Array" are commercially available.

### SUMMARY OF THE INVENTION

Here the analysis of microarray expression profiles of sigmoid colon mucosal biopsies from IBS patients and healthy subject control subjects is reported. This analysis revealed a number of differentially expressed genes in IBS patients that point to functional alterations of specific components of the host defense system and the immune response. This is in support of an important role for peripheral gastrointestinal changes underlying the aetiology of IBS. Two gene probe sets with the most strikingly increased expression in mucosal colon biopsies of IBS patients represent a gene that is, as yet, uncharacterised (DKFZP564O0823) [SEQ ID NO: 15]. It is proposed to rename this gene IBS1. IBS1 is annotated with SEQ ID NO:15 througout the specification to and refers to the gene with SEQ ID NON:15 or the protein encoded by SEQ SEQ ID NO:15 The identification of specific sets of gene probes on the microarray, so-called molecular signatures that enable the distinction of IBS patients from healthy control subjects is described. The expression profiles in IBS are consistent across different locations within the colon and are stable over time. Therefore, the identified molecular signatures provide the opportunity to develop biomarkers that are of use in the diagnosis and assessment of the response to therapy in (subsets of) IBS patients. This represents a significant advance based on specific changes in biological activities rather than the current standard, which depends exclusively on the change in clinical symptoms only.

The present invention is based on the surprising finding that, despite the absense of structural abnormalities in colon of IBS patients, it is not only possible to identify differentially expressed genes compared to normal tissue but that genes of which the differential expression is associated with IBS have a diagnostic, predictive and/or therapeutic value.

Accordingly, the present invention relates to the identification of a number of genes that were hitherto not associated with IBS, hereinafter referred to as IBS molecular signature genes (IBS-MSGs) (Table 1), and accordingly provides nucleic acid molecules in diagnostic methods to identify and IBS in a subject.

The nucleic acid molecules can be used individually, *e.g*. to monitor the level of expression of an individual gene, or they can be provided in a microarray format, to identify and/or monitor IBS in a subject. The nucleic acid molecules can be used to design antisense oligonucleotides and short-interferring RNA (siRNA), ribozymes and other molecules useful for modifying gene expression, for diagnostic, screening and therapeutic purposes. Furthermore, the nucleic acid molecules can be used to express the encoded proteins. One skilled in the art can also design peptide antigens based on the nucleic acid sequences.

In one aspect, the present invention provides methods, more particularly *in vitro* methods, for diagnosing and CVH and in particular IBS, by comparing the expression levels of one or more of the IBS-MSGs at the nucleotide level in biological samples from a subject to control samples.

Methods are disclosed for detecting and/or monitoring IBS in a subject, which methods comprise the steps of (a) determining, in a biological sample of said subject, the level of gene transcription of an IBS-MSG as claimed; (b) comparing the level of gene transcription with the level of gene transcription in a normal control sample; and (c) producing a diagnosis based on the result from step (b). More particularly the methods involve determining that there is a difference in expression of these genes compared to expression of these genes in a control sample, whereby a difference in expression in one or more of these genes is indicative of IBS, the status of IBS and/or the susceptibility to a specific type of treatment.

The methods of the present invention involve determining the increase and/or decrease of expression of particular genes. These methods involve determining whether there is an increase corresponding to at least a 1.15, more particularly at least a 1.2 fold change or whether there is a decrease corresponding to at least a 0.85 fold change, more particularly at least an 0.8 fold change in expression of the gene compared to controls.

In step (a) of the methods of the invention the level of gene transcription is determined at the gene transcription level, preferably using probes that specifically bind to an oligonucleotide transcribed from said IBS-MSG, preferably at the cDNA or mRNA level. The level of gene transcription is optionally determined using array technology, either at the oligonucleotide level using specific probes as described herein.

In specific embodiments of the methods of the invention, the level of gene transcription is assessed using an array of oligonucleotide probes that bind to the IBS-MSGs.

Typically the level of gene transcription is assessed using reverse-transcription quantitative polymerase chain reaction (RTQ-PCR).

Biological sample which are used in the methods of the present invention are blood (including total blood, serum, plasma and in particular white blood cells), urine, saliva, fecal sample, fecal cells, tissue biopsy (in particular colon biopsy) or autopsy material.

In methods of the invention the level of gene expression is determined using a array of oligonucleotide probes that bind to the IBS-MSGs as claimed,

Yet another aspect of the present invention provides kits for diagnosing CVH, in particular IBS, which kits comprises polynucleotide probes that specifically binds to an IBS-MSG.

The above and other characteristics, features and advantages of the present invention will become apparent from the following detailed description, taken in conjunction with the accompanying drawings, which illustrate, by way of example, the principles of the invention.. The reference figures quoted below refer to the attached drawings.

### BRIEF DESCRIPTION OF THE DRAWINGS

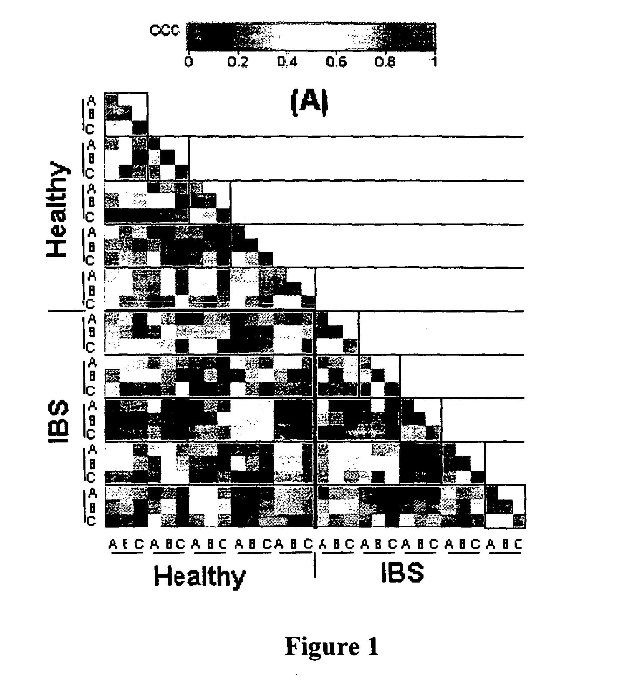

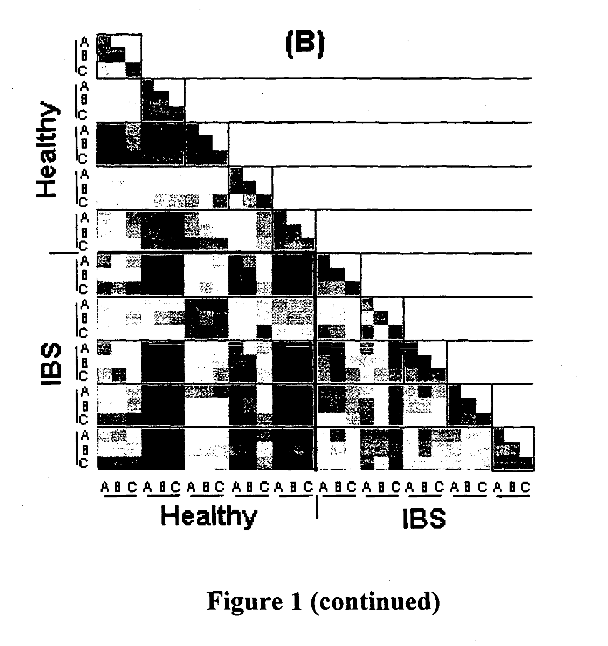

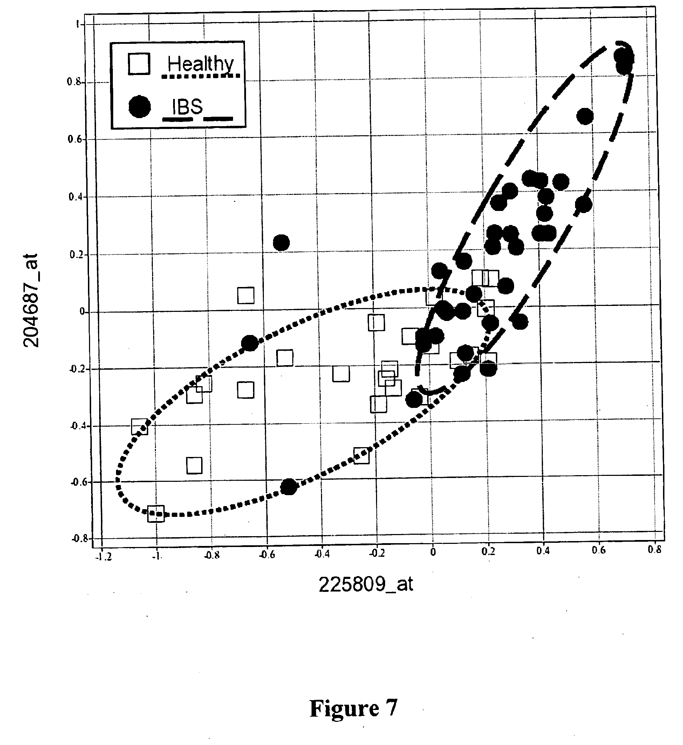

- Figure 1:: Concordance correlation analysis of the expression profiles of sigmoid colon samples collected from 10 individuals.
The degree of similarity (concordance correlation coefficient, CCC) of the samples is indicated by color codes. The analysis included three samples for each subject : sample A and B, taken at the same time, located approximately 10 cm away from each other in the colon, and sample C collected 85 days later. Blue squares indicate the concordance correlation analysis for samples from one individual. A thick black line distinguishes the samples from IBS and healthy subjects. The analysis was performed on (A) a set of 1,000 gene probes that showed the largest variation in expression within the full dataset and (B) the set of 32 gene probes identified in the prediction analysis for microarrays.
- Figure 2:: Plots representing the results of a spectral map analysis on the microarray data from the sigmoid colon samples of IBS patients and healthy subjects. The different graphs show different combinations of the six first principal components (PC) in the analysis. Red circles represent samples from subjects who volunteered for a repeat mucosal sample; blue circles represent all other subjects (no repeat sample). The subjects who provided a repeat sample did not cluster in any of these graphs, suggesting that they were indeed representative of the entire cohort.
- Figure 3:: Schematic overview of the genes differentially expressed in mucosal colon of IBS patients.
Genes with increased (underlined) or decreased expression in mucosal colon samples from IBS patients versus healthy controls. Protein complexes responsible for oxidative burst (2O₂ → 2O-) are shown as pentagon shapes. "ROS" represent Reactive Oxygen Species
- Figure 4:: Relative expression levels of significant genes identified by the Significance of Microarray Analysis (SAM) in mucosal colon samples from IBS patients (green dots) as compared to healthy controls (red dots). Expression levels (y-axis) are expressed as fluorescent signal intensity measured on the array after preprocessing of the raw data (see Methods). Each individual dot represents the averaged expression value of two samples per subject.
- Figure 5:: Gene expression profile of DKFZP564O0823 [SEQ ID NO: 15] (IBS1) and comparative sequence analysis.
(A) Gene expression profiles of two different probe sets (204678_at and 225809_at) on the Affymetrix array that represent DKFZP564O0823 [SEQ ID NO: 15] (IBS1) in mucosal colon biopsies from IBS patients (green, on the right in the figures) and healthy subjects (red, on the left in the figures). Each circle represents the average of two samples from one individual. Expression levels are expressed as signal intensity measured on the array after preprocessing of the raw data (see Methods). Green and red horizontal lines represent mean expression levels in healthy and IBS subjects, respectively. (B) This figure shows the excellent correlation between the two probesets that represent the IBS 1 gene [SEQ ID NO: 15] . Healthy controls and IBS patients are indicated as blank squares and black circles, respectively (see also Figure 7).
(C) Comparative protein sequence analysis of human (Hs) DKFZP564O0823 [SEQ ID NO: 15] (IBS1) (Hs_NP_056208) and its mouse (Mm_NP_663537) and rat (Rn_NP_775137) homologues. Identical amino acids over different species are highlighted with a black or grey background. Different domains of the protein are indicated under the amino acid sequence.

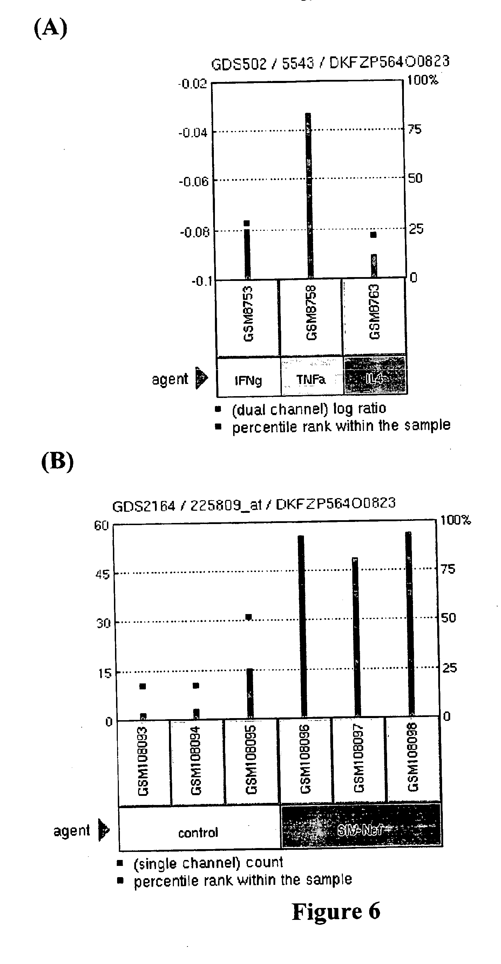

- Figure 6:: (A) Examination of DKFZP564O0823 gene expression induced by interferon gamma (IFNγ), tumor necrosis factor alpha (TNFα) and interleukin 4 (IL4) inflammatory cytokines on primary colon endothelial cells. From: Gene Expression Omnibus, Accession nr. GDS502 (http://www.ncbi.nlm.nih.gov/projects/geo/gds/profileGraph.cgi?&dataset= MzA&dataset=S40$&gmin=-0.090309&gmax=-0.032624&gds=502&idref =5543&annot=DKFZP564O0823)
(B) Analysis of Jurkat CD4+ T cells following induction of simian immune deficiency virus (SIV) Nef from an inducible promoter. The Nef protein is expressed early in HIV and SIV infections and plays a crucial role in disease progression. Results identify Nef-mediated changes in T cell gene expression. From: Gene Expression Omnibus, Accession nr. GDS2164 (http://www.ncbi.nlm.nih.gov/projects/geo/gds/profileGraph.cgi?&dataset= ABPyqz&dataset=LL6gee$&gmin=2.600000&gmax=56.900000&gds=2164 &idref=225809_at&annot=DKFZP564O0823)
- Figure 7:: Correlation of the expression levels of two probe sets, 204687_at and 225809_at, representing the DKFZP564O0823 gene. Healthy controls and IBS patients are indicated as blank squares (grouped by a dotted line) and black circles (grouped by a dashed line), respectively.

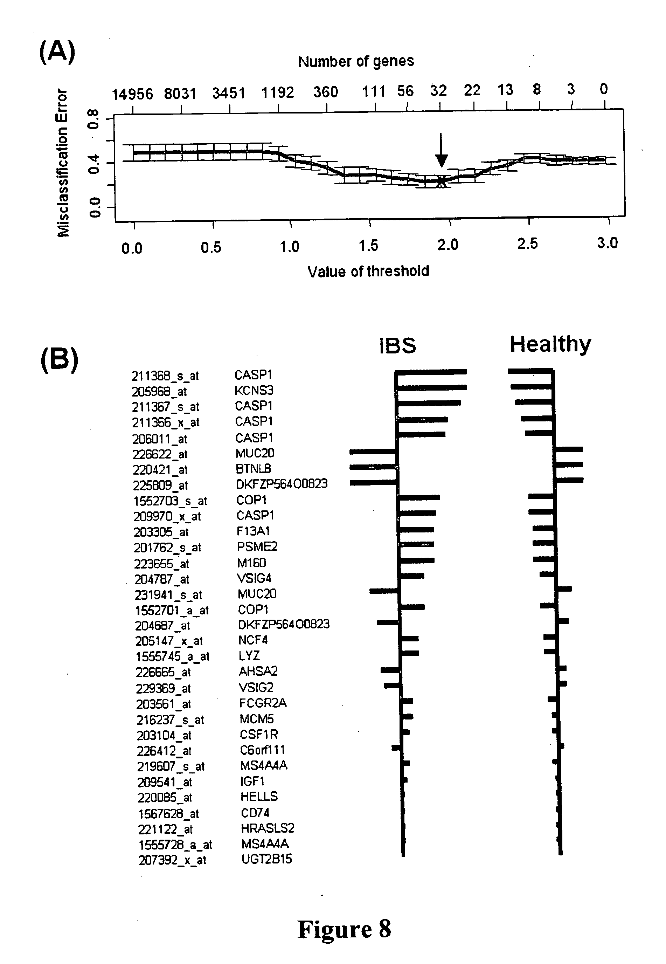

- Figure 8:: Summary of the Predictive Analysis of Microarrays (PAM): output from the nearest shrunken centroid classifier on IBS disease status.
(A) The cross-validated misclassification error curve that shows that the lowest misclassification error is obtained when using 32 genes. The corresponding delta (2.0) was selected as threshold for further analysis.
(B) The shrunken class centroids for each class for the 32 genes surviving the threshold (delta =2.0). For more details, see Tibshirani et al (2002).

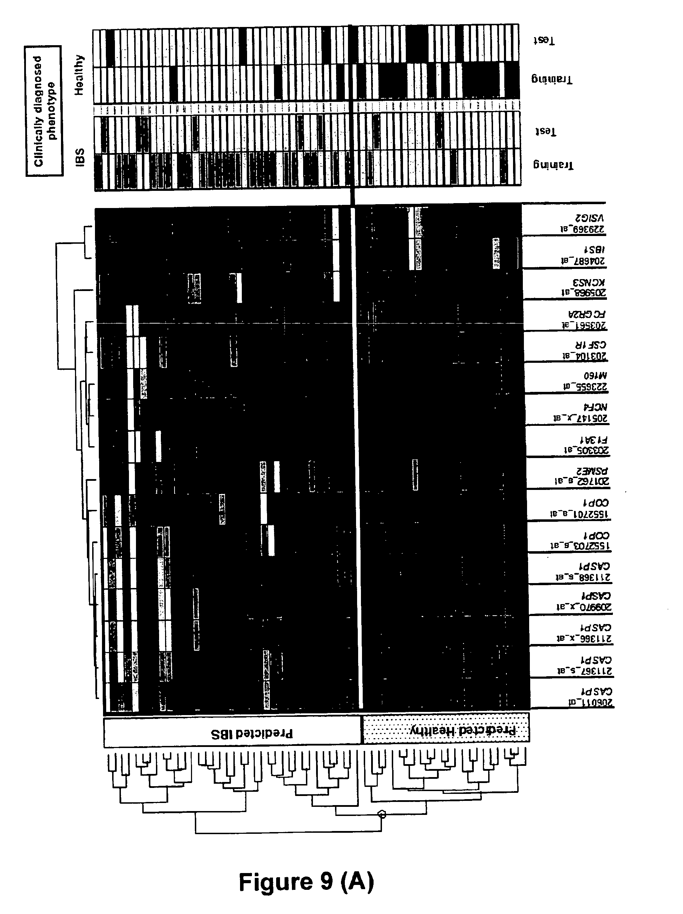

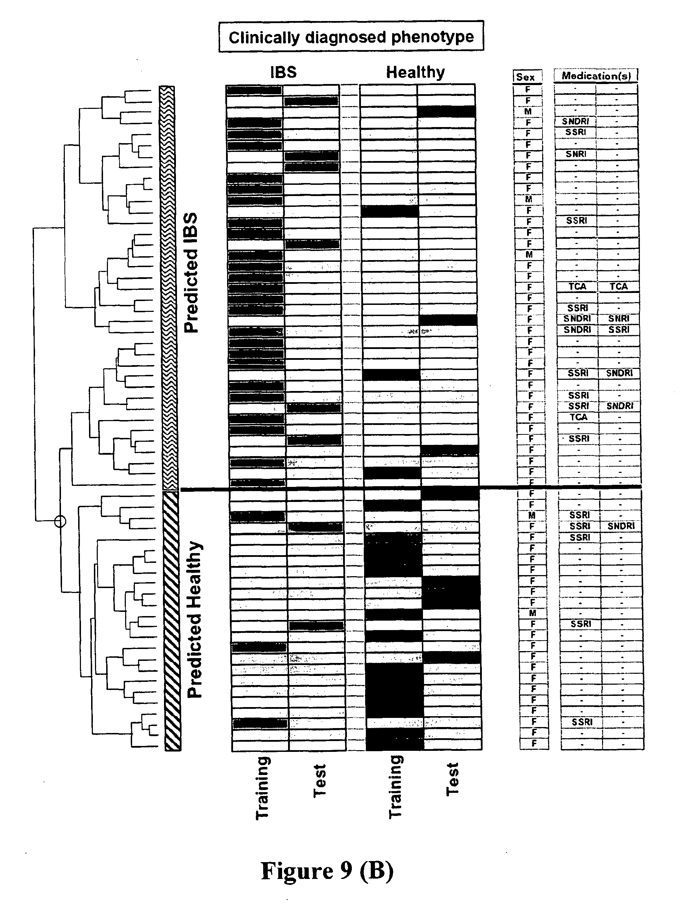

- Figure 9:: Summary of hierarchical clustering analysis.
(A) Clustered display of heatmap with hierarchical clustering of 16 probesets and samples using average linkage and correlation as similarity measure. The colors of the heatmap represent the relative expression level on a color gradient scale ranging from blue (high expression) to black (intermediate expression) to yellow (low expression). This color scale is maximized for each individual probeset over all the samples (i.e., the sample with the highest expression level is blue, and the sample with the lowest expression is yellow). The white horizontal line on the heat map discriminates the disease status as predicted by the obtained molecular signature for IBS. The right panel of the figure shows the clinically diagnosed disease status in the subjects assigned to the training or the test set.
(B) The right panel of figure 9A is shown again, now also linked to the gender of the subjects and concomitant drug treatment. (M: male; F: female; SSRI: selective serotonin reuptake inhibitor; SNRI: serotonin-norepinephrine reuptake inhibitor; SNDRI: serotonin-norepinephrine-dopamine reuptake inhibitor; TCA: tricyclic antidepressant).

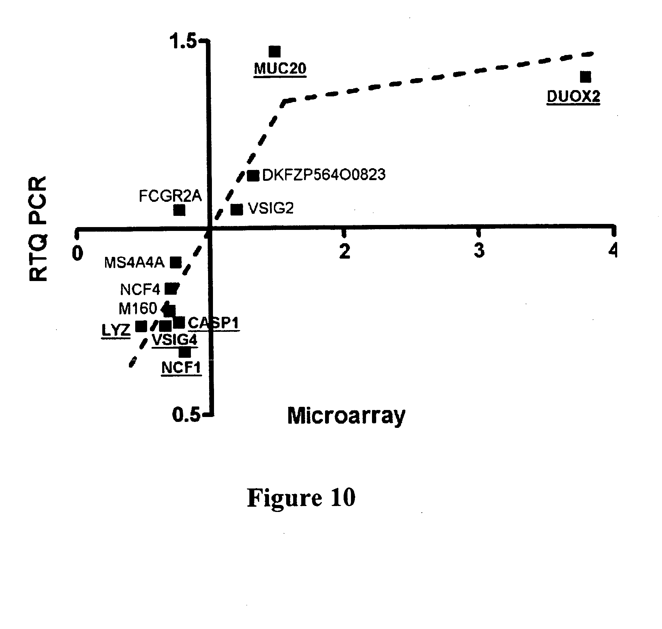

- Figure 10:: Comparison of fold changes in mRNA expression level, as measured by microarray and RTQ-PCR, between IBS patients and healthy subjects. Significant genes from the microarray study that were confirmed statistically significant (p<0.05) in RTQ-PCR analysis are underlined.

### DETAILED DESCRIPTION

The preferred embodiments of the invention are described below. Unless specifically noted, it is intended that the words and phrases in the specification and claims be given the ordinary and accustomed meaning to those of ordinary skill in the applicable art of arts. If any other meaning is intended, the specification will specifically state that a special meaning is being applied to a word or phrase.

It is further intended that the inventions not be limited only to the specific structure, material or acts that are described in the preferred embodiments, but in addition, include any and all structures, materials or acts that perform the claimed function, along with any and all later-developed equivalent structures, materials or acts for performing the claimed invention.

Further examples exist throughout the disclosure, and it is not applicant's intention to exclude the use of structures, materials, methods or acts that are not expressely identified in the specification, but nonetheless are capable of performing a claimed function.
As used herein, the term "compound" or "agent" means a biological or chemical compound such as a simple or complex organic molecule, a peptide, a protein or an oligonucleotide. A "test compound" as used herein, refers to a "compound" or "agent" used to assess whether said compounds binds with and/or modulates an activity of an IBS-MSG product.
The term "chronic visceral hypersensitivity-molecular signal genes" or "CVH-MSG" as used herein refers to genes, the expression of which is associated with the clinical diagnosis of chronic visceral hypersensitivity (CVH). This includes genes which are specifically upregulated or downregulated in patients diagnosed with CVH compared to healthy control patients.
The term "inflammatory bowel syndrome molecular signature genes" or "IBS-MSG" as used herein refers to genes, the expression of which is associated with the clinical diagnosis of inflammatory bowel syndrome (IBS). This includes genes which are specifically upregulated or downregulated in patients diagnosed with IBS compared to healthy control patients.
The term "IBS-MSG product" or "gene product" as used herein includes a polynucleotide or polypeptide and variants thereof, generated when an IBS-MSG is transcribed and/or translated.
As used herein, a "variant of a polynucleotide" includes a polynucleotide that differs from the original polynucleotide by one or more substitutions, additions, deletions and/or insertions such that the activity of the encoded polypeptide is not substantially changed (*e.g*., the activity may be diminished or enhanced, by less than 50%, and preferably less than 20%) relative to the polypeptide encoded by the original polynucleotide.
   A variant of a polynucleotide also includes polynucleotides that are capable of hybridizing under reduced stringency conditions, more preferably stringent conditions, and most preferably highly stringent conditions to the original polynucleotide (or a complementary sequence).
   It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotide where alterations are limited to silent changes, *i.e*. changes that do not alter the amino acids encoded by the polynucleotide are specifically contemplated by the present invention.
   Polynucleotide variants preferably exhibit at least about 70%, preferably at least 80%, more preferably at least 90%, even more preferably at leat 95%, in particular at least 97%, and most preferably at least 99% sequence homology with the native polynucleotide.
The term "hybridization" as used herein refers to a process in which a single-stranded nucleic acid molecule joins with a complementary strand through nucleotide base pairing.
The term "stringency" refers to hybridization conditions. High stringency conditions disfavor non-homologous base pairing. Low stringency conditions have the opposite effect. Stringency may be altered, for example, by temperature and salt concentration. "Stringent conditions" refer to an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denaturated, sheared salmon sperm DNA, followed by washing the filters in 0.1 x SSC at about 65°C. Further suitable hybridization conditions are described in the examples.
"Lower stringency conditions" include an overnight incubation at 37°C in a solution comprising 6x SSPE (20x SSPE = 3M NaCl; 0.2M NaH₂PO₄; 0.02M EDTA, pH 7.4), 0.5% SDS, 30% formamide,100 µg/ml salmon sperm blocking DNA; followed by washes at 50°C with 1 X SSPE, 0.1% SDS. In addition, to achieve even lower stringency, washes performed following stringent hybridization can be done at higher salt concentrations (e. g. 5X SSC). Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.
The terms "complementary" or "complementarity" as used herein refer to the capacity of purine and pyrimidine nucleotides to associate through hydrogen bonding to form double-stranded nucleic acid molecules. The following base pairs are related by complementarity: guanine and cytosine; adenine and thymine; and adenine and uracil. As used herein "complementary" means that the aforementioned relationship applies to substantially all base pairs comprising two single-stranded nucleic acid molecules over the entire length of said molecules. "Partially complementary" refers to the aforementioned relationship in which one of the two single-stranded nucleic acid molecules is shorter in length than the other such that a portion of one of the molecules remains single-stranded.
The term "subject" as used herein refers to a mammal (*e.g*., a rodent such as a mouse or a rat, a pig, a primate, or a companion animal (*e.g*., dog or cat)). In particular, the term refers to humans.
The terms "array" and "microarray" are used interchangeably and refer generally to any ordered arrangement (*e.g*., on a surface or substrate) of different molecules, referred to herein as "probes". Each different probe of an array is capable of specifically recognizing and/or binding to a particular molecule, which is referred to herein as its "target," in the context of arrays. Examples of typical target molecules that can be detected using microarrays include mRNA transcripts, cDNA molecules, cRNA molecules, and proteins.
   Microarrays are useful for simultaneously detecting the presence, absence and quantity of a plurality of different target molecules in a sample (such as an mRNA preparation isolated from a relevant cell, tissue, or organism, or a corresponding cDNA or cRNA preparation). The presence and quantity, or absence, of a probe's target molecule in a sample may be readily determined by analyzing whether (and how much of) a target has bound to a probe at a particular location on the surface or substrate.
In a preferred embodiment, arrays used in the present invention are "addressable arrays" where each different probe is associated with a particular "address". For example, in a preferred embodiment where the probes are immobilized on a surface or a substrate, each different probe of the addressable array is immobilized at a particular, known location on the surface or substrate. The presence or absence of that probe's target molecule in a sample may therefore readily be determined by simply detecting whether a target has bound to that particular location on the surface or substrate.
The arrays according to the present invention are nucleic acid arrays (also referred to herein as "transcript arrays" or "hybridization arrays") that comprise a plurality of nucleic acid probes immobilized on a surface or substrate. The different nucleic acid probes are complementary to, and therefore can hybridize to, different target nucleic acid molecules in a sample. Thus, such probes can be used to simultaneously detect the presence and quantity of a plurality of different nucleic acid molecules in a sample, to determine the expression level of a plurality of different genes, e.g. the presence and abundance of different mRNA molecules, or of nucleic acid molecules derived therefrom (for example, cDNA or cRNA).
   There are two major types of microarray technology; spotted cDNA arrays and manufactured oligonucleotide arrays. The Examples section below describes the use of high density oligonucleotide Affymetrix GeneChip® arrays. The arrays are preferably reproducible, allowing multiple copies of a given array to be produced and the results from each easily compared to each other. Preferably the microarrays are small, usually smaller than 5cm, and are made from materials that are stable under binding (*e.g*. nucleic acid hybridization) conditions. A given binding site or unique set of binding sites in the microarray will specifically bind the target (*e.g*., the mRNA of a single gene in the cell). Although there may be more than one physical binding site (hereinafter"site") per specific target, for the sake of clarity the discussion below will assume that there is a single site. It will be appreciated that when cDNA complementary to the RNA of a cell is made and hybridized to a microarray under suitable hybridization conditions, the level or degree of hybridization to the site in the array corresponding to any particular gene will reflect the prevalence in the cell of mRNA transcribed from that gene. For example, when detectably labeled (*e.g*. with a fluorophore) cDNA complementary to the total cellular mRNA is hybridized to a microarray, any site on the array corresponding to a gene (*i.e.* capable of specifically binding a nucleic acid product of the gene) that is not transcribed in the cell will have little or no signal, while a gene for which the encoded mRNA is highly prevalent will have a relatively strong signal.
   By way of example, GeneChip® expression analysis (Affymetrix, Santa Clara, CA) generates data for the assessment of gene expression profiles and other biological assays.
   Oligonucleotide expression arrays simultaneously and quantitatively "interrogate" thousands of mRNA transcripts (genes orESTs), simplifying large genomic studies. Each transcript can be represented on a probe array by multiple probe pairs to differentiate among closely related members of gene families. Each probe set contains millions of copies of a specific oligonucleotide probe, permitting the accurate and sensitive detection of even low-intensity mRNA hybridization patterns. After hybridization intensity data is captured, *e.g*., using optical detection systems (*e.g*., a scanner), software can be used to automatically calculate intensity values for each probe cell. Probe cell intensities can be used to calculate an average intensity for each gene, which correlates with mRNA abundance levels. Expression data can be quickly sorted based on any analysis parameter and displayed in a variety of graphical formats for any selected subset of genes. Gene expression detection technologies include, among others, the research products manufactured and sold by Hewlett-Packard, Perkin-Elmer and Gene Logic.
The term "conservative substitution" or "conservative amino acid substitution" refers to a replacement of one or more amino acid residue(s) in a parent protein without affecting the biological activity of the parent molecule based on the art recognized substitutability of certain amino acids (See e.g. M. Dayhoff, In Atlas of Protein Sequence and Structure, Vol. 5, Supp. 3, pgs 345-352, 1978).
"Fragment thereof" refers to a fragment, piece, or sub-region of a nucleic acid or protein molecule whose sequence is disclosed herein, such that said fragment comprises 5 or more amino acids, or 10 or more nucleotides that are contiguous in the parent protein or nucleic acid molecule.
"Functional fragment" as used herein, refers to an isolated sub-region, or fragment of a protein disclosed herein, or sequence of amino acids that, for example, comprises a functionally distinct region such as an active site for a receptor. Functional fragments may be produced by cloning technology, or as the natural products of alternative splicing mechanims.
The term "homolog" or "homologous" describes the relationship between different nucleic acid molecules or amino acid sequences in which said sequences or molecules are related by partial identity or similarity at one or more blocks or regions within said molecules or sequences. "Isolated nucleic acid compound" refers to any RNA or DNA sequence, however construed or synthesized, which is locationally distinct from its natural location.
As used herein "identity or similarity", as known in the art, are relationships between two or more polypeptide sequences or two or more polynucleotide sequences, as determined by comparing the sequences. In the art, identity also means the degree of sequence relatedness between polypeptide or polynucleotide sequences, as the case may be, as determined by the match between strings of such sequences. Both identity and similarity can be readily calculated (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exist a number of methods to measure identity and similarity between two polynucleotide or two polypeptide sequences, both terms are well known to skilled artisans (Sequence Analysis in Molecular Biology, von Heinje, G., Academic Press, 1987; Sequence Analysis Primer, Gribskov, M. and Devereux, J., eds., M Stockton Press, New York, 1991; and Carillo, H., and Lipman, D., (1988) SIAM J. Applied Math., 48, 1073. Methods commonly employed to determine identity or similarity between sequences include, but are not limited to those disclosed in Carillo, H., and Lipman, D., (1988) SIAM J. Applied Math., 48, 1073.
   Methods for comparing the identity and similarity of two or more sequences are well known in the art. Thus for instance, programs available in the Winconsin Sequence Analysis Package, version 9.1 (Devreux J. et al., Nucleic Acid Res., 12, 387-395, 1984), for example the programs BESTFIT and GAP, may be used to determine the % identity between two polynucleotides and the % identity and the % similarity between two peptide or polypeptide sequences. BESTFIT uses the "local homology" algorithm of Smith and Waterman (J. Mol. Biol., 147, 195-197, 1981) and finds the best single region of similarity between two sequences. BESTFIT is more suited to compare two polynucleotide or two peptide or polypeptide sequences that are dissimilar in length, the program assuming that the shorter sequence represents a portion of the longer. In comparison, GAP aligns two sequences, finding a "maximum similarity", according to the algorithm of Needleman and Wunsch (J.Mol.Biol., 48, 443-453, 1970). GAP is more suited to compare sequences that are approximately the same length and an alignment is expected over the entire length. Preferably, the parameters "Gap Weight" and "Length Weight" used in each program are 50 and 3, for polynucleotide sequences and 12 and 4 for polypeptide sequences, respectively. Preferably, % identities and similarities are determined when the two sequences being compared are optimally aligned. Other programs for determining identity and/or similarity between sequences are also known in the art, for instance the BLAST family of programs (Altschul S F et al., Nucleic Acids Res., 25:3389-3402, 1997).
A "nucleic acid probe" or "probe" as used herein is a nucleic acid compound, in particular a labeled nucleic acid compound, which hybridizes with another nucleic acid compound. "Nucleic acid probe" means a single stranded nucleic acid sequence that will hybridize with a single stranded target nucleic acid sequence. A nucleic acid probe may be an oligonucleotide or a nucleotide polymer. A "probe" will usually contain a detectable moiety which may be attached to the end(s) of the probe or be internal to the sequence of the probe. In a specific embodiment "probe" is also used to refer to an oligonucleotide, for example about 25 nucleotides in length, attached to a solid support for use on "arrays" and "microarrays" as described hereinbefore.
The term "primer" is a nucleic acid fragment which functions as an initiating substrate for enzymatic or synthetic elongation of, for example, a nucleic acid molecule.
The term "hybridization" as used herein refers to a process in which a single-stranded nucleic acid molecule joins with a complementary strand through nucleotide base pairing.
As used herein, the term "modulation" includes in its various grammatical forms (*e.g.* "modulated", "modulation", "modulating", etc.), up-regulation, induction, stimulation, potentiation and/or relief of inhibition, as well as inhibition and/or down regulation or suppression.
A nucleic acid sequence is "operably linked" to another nucleic acid sequence when the former is placed into a functional relationship with the latter. For example, a DNA for a presequence or secretory leader peptide is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However, enhancers do not have to be contiguous. Linking is accomplished by ligation at convenient restriction sites. If such sites do not exist, synthetic oligonucleotide adaptors or linkers are used in accordance with conventional practice.

The present invention is based on the identification of a number of genes which are associated with the clinical symptoms of CVH, more particularly with IBS. These genes have been identified by differential expression analysis of patients diagnosed with IBS and healthy controls. More particularly, the diagnosis of patients with IBS by a gastroentereologist was confirmed by the a bowel disease questionnaire (Talley et al., 1990) including questions to correspond to Rome II criteria (Thompson et al., 1999). The bowel disease questionnaire also includes a psychosomatic symptom checklist intended to identify somatization disorders and symptoms to characterize non-ulcer dyspepsia, and has been used extensively in epidemiological studies.

According to the present invention, genes have been identified the expression of which in mucusal colon is either decreased or increased in patients with CVH, more particularly, IBS, compared to healthy controls. A particular advantage of these expression markers is that there is a strong correlation between the expression of particular genes and the occurrence of IBS, and that these expression patterns have a predictive value. Accordingly, these expression markers are useful as a diagnostic tool. Expression markers are not necessarily, and even in most cases, not linked to the presence of a polymorphism in the corresponding genes, distinguishing them from genetic markers.

### Screening methods

The present invention discloses nucleic acid molecules and gene products (proteins) that can be used in screening assays to identify compounds for use as therapeutics for the treatment of CVH, in particular IBS.
The proteins encoded by the nucleic acid molecules described herein can be used in binding and functional assays to screen for lead compounds for treating CVH, in particular IBS. As discussed for each gene product, the ability to identify either an antagonist or agonist would provide for development of new treatments for IBS

Thus, the nucleic acid molecules are useful for expressing the proteins to be isolated and used in direct binding assays. Protein expression can be carried out in any host cell system, e. g, plants, prokaryotes (e.g. E. coli), yeast, insect cells (e.g. Sf9 cells, using baculovirus vectors), or mammalian cells (e.g. CHO, COS etc.). Techniques for the isolation and purification of the protein products are well known to one skilled in the art.

Protein products, or fragments thereof (*e.g*. proteolytic fragments or synthetic fragments), can be used to generate specific antibodies for directly detecting protein expression, e. g. through immunoassay.

Gene expression profiles may be used in screening for compounds that modulate the mRNA or protein expression of the differentially expressed genes shown in Table 1. Such a differentially expressed gene is referred to as the "gene of interest" and such modulating compounds are referred to as modulators that may up- or down-regulate mRNA transcription, or agonize or antagonize the activity of the protein. Such compounds are useful, *e.g*. for inhibiting or stimulating the expression of genes found to be regulated in IBS. Compounds that modulate the expression profile of one or more of the genes may be readily identified using numerous screening methods known in the art. As used herein, the expression of a gene can be determined by measuring mRNA levels, protein levels, or protein activity using standard techniques.

Methods are provided for identifying a candidate compound for the treatment of CVH, in particular for the treatment of IBS, said method comprising;
a) contacting a cell expressing at least one IBS-MSG with the compound to be tested;
b) determining the expression level of said IBS-MSG; and
c) comparing with the expression level of said IBS-MSG in the absence of said compound;
whereby a compound capable of opposing the change in expression level of the IBS-MSG observed in IBS, is identified as a candidate compound for the treatment of CVH, in particular for the treatment of IBS.
The IBS-MSG as used in the screening methods is typically selected from the group consisting of IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4 and MUC20; in particular from the group consisting of IBS1 [SEQ ID NO: 15], COP1, PSME2, F13A1, NCF4, CSF1R, M160, KCNS3, VSIG2; more in particular from the group consisting of IBS1 [SEQ ID NO: 15] , PSME2, F13A1, NCF4, CSFR1 AND VSIG2; even more in particular from the group consisting of MUC20, VSIG2 and VSIG4; most particular the IBS-MSG used in the screening methods of the present invention consists of IBS1 [SEQ ID NO: 15] .
, the cell is a colon cell, more particularly, a mucosal colon cell.
For example the methods comprise, in step (b) determining the expression of at least two different genes, one of which is selected from the group consisting of IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4, MUC20 and one or more other genes is selected from the group consisting of IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4, MUC20, CASP1, FCGR2A and CKB; in particular determining the expression of at least two genes selected from the group consisting of IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4 and MUC20; in particular at least two genes selected from the group consisting of IBS1 [SEQ ID NO: 15], COP1, PSME2, F13A1, NCF4, CSF1R, M160, KCNS3, VSIG2; more in particular at least two genes from the group consisting of IBS1 [SEQ ID NO: 15], PSME2, F13A1, NCF4, CSFR1 AND VSIG2; even more in particular at least two genes from the group consisting of MUC20, VSIG2 and VSIG4; further examples comprise in step (b) determining the expression of at least two genes, one of which is IBS1 [SEQ ID NO: 15], the other being selected from the group consisting of COP1, PSME2, F13A1, NCF4, CSFR1, M160, KCNS3, LYZ, MS4A4A, HELLS, FRC4, MCM5, TAP2, LRAP, DTL, VSIG2, VSIG4, MUC20, CASP1, FCGR2A and CKB.
For each of the embodiments described thereof, step (c) consists of comparing the expression level of said at least two genes by said cells after having contacted said cell with said compound to the expression of said genes by said cells in the absence of said compound.

As used herein, the expression level of an IBS-MSG can be detected at the nucleic acid level or at the protein level. Determining the expression level at the nucleic acid level can be accomplished using any available technology to measure gene transcription levels. For example, the method could employ *in situ* hybridization, Northern hybridization or hybridization to a nucleic acid microarray, such as an oligonucleotide microarray or a cDNA microarray. Alternatively, the method could employ reverse-transcriptase polymerase chain reaction (RT-PCR) such as fluorescent dye-based quantitative real time PCR (TaqMan^{®} PCR). In the example section provided below, nucleic acid expression levels were obtained by hybridization of labeled cRNA derived from total cellular mRNA to Affymetrix GeneChip^{®} oligonucleotide microarrays and using RTQ-PCR (TaqMan^{®} PCR). The expression levels at the protein level can be assessed using any available technology to measure protein levels. For example, the method could employ protein microarray technology, Western blotting, immunocytochemistry, SDS-PAGE, relative quantification using mass spectrometry and pre-labelling of cells with isotopomeric forms of essential amino acids (Unwin R.D., Evans, C.A. and Whetton A.D. 2006 TRENDS in Biochemical Sciences Vol.31(8); 473-484).

Preferably, the expression level is determined at the nucleic acid level. In this instance mRNA or cDNA may be used directly for detection or may be amplified enzymatically using PCR or other amplification techniques prior to analysis. Preferably said analysis methods comprise the use of a labelled oligonucleotide probe targeted to a suitable region of the polynucleotide expression level is determined using a probe which binds to an IBS-MSG as claimed. Oligonucleotide probes are disclosed in Table 1 and Table 2 below.

**TABLE 2**

| **DESCRIPTION OF THE PROBE SETS** | |
|---|---|
| **CASP1 specific probes** | |
| caspase 1, apoptosis-related cysteine peptidase (interleukin 1, beta, convertase) | |
| Refseq ID (NCBI): | NM_001223 |
| SwissProt: | P29466 |
| Refseq protein ID (NCBI): | NP_001214.1 |
| | |

| **SEQ ID No.1** | |
|---|---|
| 206011_at | CASP1 |
| | |
| >HG-U133_PLUS_2:206011_AT | |

| | |
|---|---|
| Genbank: | AI719655 (nt 1539-1970) |

| **SEQ ID No.2** | |
|---|---|
| 211367_s_at | CASP1 |
| >HG-U133_PLUS_2:211367_S_AT | |
| | |

| | |
|---|---|
| Genbank: U13699 | (nt 275-561) |

| **SEQ ID No.3** | |
|---|---|
| 211366_x_at | CASP1 |

| | |
|---|---|
| >HG-U133_PLUS_2:211366_X_AT | |

| | |
|---|---|
| Genbank: | U13698 (nt 402-925) |

| **SEQ ID No.4** | |
|---|---|
| 209970_x_at | CASP1 |
| >HG-U133_PLUS_2:209970_X_AT | |

| | |
|---|---|
| Genbank: | M87507 (nt 859-1133) |

| **SEQ ID No.5** | |
|---|---|
| 211368_s_at | CASP1 |
| >HG-U133_PLUS_2:211368_S_AT | |

| | |
|---|---|
| Genbank: | U13700 (nt73-258) |
| | |

| **COP1 specific probes** | |
|---|---|
| caspase-1 dominant-negative inhibitor pseudo-ICE | |
| Refseq ID (NCBI): | NM_001017534 |
| SwissProt: | Q5EG05 |
| Refseq protein ID (NCBI): | NP_001017534.1 |
| | |

| **SEQ ID No.6** | |
|---|---|
| 1552703_s_at | COP1 |
| | |
| >HG-U133_PLUS_2:1552703_S_AT | |

| | |
|---|---|
| Genbank: | NM_052889 (nt 86-267) |

| **SEQ ID No.7** | |
|---|---|
| 1552701_a_at | COP1 |
| >HG-U133_PLUS_2:1552701_A_AT | |
| aggtccgatacctggaaattagcttagtacacaagactcccaattactattttct | |
| Genbank: NM_052889 | (nt 314-344) |

| **PSME2 specific probe** | |
|---|---|
| proteasome (prosome, macropain) activator subunit 2 (PA28 beta) | |
| Refseq ID (NCBI): | NM_002818 |
| SwissProt: | Q2TNB3 |
| Refseq protein ID (NCBI): | NP_002809.2 |

| **SEQ ID No.8** | |
|---|---|
| 201762_s_at | PSME2 |
| >HG-U133_PLUS_2:201762_S_AT | |

| | |
|---|---|
| Genbank: NM_002818 | (nt 453-723) |

| **F13A1 specific probe** | |
|---|---|
| coagulation factor XIII, A1 polypeptide | |
| Refseq ID (NCBI): | NM_000129 |
| SwissProt: | P00488 |
| Refseq protein ID (NCBI): | NP_000120.1 |

| **SEQ ID No.9** | |
|---|---|
| 203305_at | F13A1 |
| >HG-U133_PLUS_2:203305_AT | |

| | |
|---|---|
| Genbank: | NM_000129 (nt 3289-3718) |

| **==========** **NCF4 specific probe** | |
|---|---|
| neutrophil cytosolic factor 4, 40kDa | |
| Refseq ID (NCBI): | M_000631 |
| SwissProt: | Q15080 |
| Refseq protein ID (NCBI): | NP_000622.2 |
| | |

| **SEQ ID No.10** | |
|---|---|
| 205147_x_at | NCF4 |
| | |
| >HG-U133_PLU&S_2:205147_X_AT | |

| | |
|---|---|
| Genbank: M_000631 | (nt 690-1162) |

| **==========** **M160 specific probe** | |
|---|---|
| Scavenger receptor cysteine-rich type 1 protein M160, precursor (CD 163 molecule-like 1) | |
| Refseq ID (NCBI): | NM_174941 |
| SwissProt: | Q2M3B7 |
| Refseq protein ID (NCBI): | NP_777601.2 |

| **SEQ ID No.11** | |
|---|---|
| 223655_at | M160 (CD163L1) |
| | |
| >HG-U133_PLUS_2:223655_AT | |

| | |
|---|---|
| Genbank: NM_174941 | (nt 4000-4415) |

| **==========** **CSF1R specific probe** | |
|---|---|
| colony stimulating factor 1 receptor | |
| Refseq ID (NCBI): | NM_005211 |
| SwissProt: | P07333 |
| Refseq protein ID (NCBI): | NP_005202.2 |

| **SEQ ID No.12** | |
|---|---|
| 203104_at CSF1R | |
| >HG-U133_PLUS_2:203104_AT | |
| | |

| | |
|---|---|
| Genbank: | NM_005211 (nt 3485-3942) |

| **==========** **FCGR2A specific probe** | |
|---|---|
| Fc fragment of IgG, low affinity IIa, receptor (CD32) | |
| Refseq ID (NCBI): | NM_021642 |
| SwissProt: | P12318 |
| Refseq protein ID (NCBI): | NP_067674.2 |

| **SEQ ID No.13** | |
|---|---|
| 203561_at | FCGR2A |
| | |
| >HG-U133_PLUS_2:203561_AT | |

| | |
|---|---|
| Genbank: NM_021642 | (nt 1710-2200) |
| | |

| **==========** **KCNS3 specific probe** | |
|---|---|
| potassium voltage-gated channel, delayed-rectifier, subfamily S, member 3 | |

| | |
|---|---|
| Refseq ID (NCBI): | NM_002252 |
| SwissProt: | Q9BQ31 |
| Refseq protein ID (NCBI): | NP_002243.3 |
| | |

| **SEQ ID No.14** | |
|---|---|
| 205968_at | KCNS3 |
| | |
| >HG-U133_PLUS_2:205968_AT | |

| | |
|---|---|
| Genbank: NM_002252 | (nt 1521-1973) |

| **==========** **IBS1 specific probe** | |
|---|---|
| DKFZP564O0823 protein | |
| Refseq ID (NCBI): | NM_015393 |
| SwissProt: | Q6UWI2 |
| Refseq protein ID (NCBI): | NP_056208.2 |

| **SEQ ID No.15** | |
|---|---|
| 204687_at | DKFZP564O0823 (IBS1) |
| >HG-U133_PLUS_2:204687_AT | |

| | |
|---|---|
| Genbank: NM_015393 | (nt 1602-2089) |

| **==========** **VSIG2 specific probe** | |
|---|---|
| V-set and immunoglobulin domain containing 2 | |
| Refseq ID (NCBI): | NM_014312 |
| SwissProt: | Q96IQ7 |
| Refseq protein ID (NCBI): | NP_055127.2 |

| **SEQ ID No.16** | |
|---|---|
| 229369_at | VSIG2 |
| >HG-U133_PLUS_2:229369_AT | |

| | |
|---|---|
| Genbank: AI201858 | (nt 940-1143) |

Alternatively, the level of gene transcription is determined at protein level and the present disclosure provides a method for identifying a candidate compound for the treatment of CVH, in particular for the treatment of IBS, said method comprising;
a) contacting a cell expressing at least one IBS-MSG with the compound to be tested;
b) determining the protein level of said IBS-MSG; and
c) comparing with the protein level of said IBS-MSG in the absence of said compound;
whereby a compound capable of opposing the change in protein level of the IBS-MSG observed in IBS, is identified as a candidate compound for the treatment of CVH, in particular for the treatment of IBS.
Preferably, the protein level is determined using an antibody that binds to an IBS-MSG product. In particular using an antibody which binds to a polypeptide encoded by an IBS-MSG selected from the group consisting of IBS[SEQ ID NO: 15] 1, COP1, PSME2, F13A1, NCF4, CSFR1, M160, KCNS3, LYZ, MS4A4A, HELLS, FRC4, MCM5, TAP2, LRAP, DTL, VSIG2, VSIG4 and MUC20; in particular from the group consisting of IBS1 [SEQ ID NO: 15] , COP1, PSME2, F13A1, NCF4, CSF1R, M160, KCNS3, VSIG2; more in particular from the group consisting of IBS1 [SEQ ID NO: 15], PSME2, F13A1, NCF4, CSFR1 AND VSIG2; even more in particular from the group consisting of MUC20, VSIG2 and VSIG4; most particular the IBS-MSG used in the screening methods consists of IBS1 [SEQ ID NO: 15] .
According to a particular example, these methods comprise, in step (b) determining the determine the protein level of the gene product of at least two different genes, one of which is selected from the group consisting of IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4, MUC20 and one or more other genes is selected from the group consisting of IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4, MUC20, CASP1, FCGR2A and CKB; in particular determining the protein level of the gene product of at least two genes selected from the group consisting of IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4 and MUC20; in particular at least two genes selected from the group consisting of IBS1 [SEQ ID NO: 15], COP1, PSME2, F13A1, NCF4, CSF1R, M160, KCNS3, VSIG2; more in particular at least two genes from the group consisting of IBS1 [SEQ ID NO: 15], PSME2, F13A1, NCF4, CSFR1 AND VSIG2; even more in particular at least two genes from the group consisting of MUC20, VSIG2 and VSIG4; further examples comprise in step (b) determining the protein level of of at least two gene products, one of which is IBS1-gene [SEQ ID NO: 15] product , the other being selected from the group consisting of the gene-products of COP1, PSME2, F13A1, NCF4, CSFR1, M160, KCNS3, LYZ, MS4A4A, HELLS, FRC4, MCM5, TAP2, LRAP, DTL, VSIG2, VSIG4, MUC20, CASP1, FCGR2A and CKB.

Antibodies generated against polypeptides as disclosed herein may be obtained by administering the polypeptides or epitope-bearing fragments, analogs or cells expressing these to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975)256:495-497), the trioma technique, the human B-cell hybridoma technique (Kozbor et al., Immunology Today (1983)4:72) and the EBV-hybridoma technique (Cole et al., MONOCLONAL ANTIBODIES AND CANCER THERAPY, pp.77-96, Alan R. Liss, Inc., 1985).

Techniques for the production of single chain antibodies, such as those described in U.S. Patent No.4,946,778, can also be adapted to produce single chain antibodies to polypeptides. Also, transgenic mice, or other organisms, including other mammals, may be used to express humanized antibodies.

The above-described antibodies may be employed to isolate or to identify clones expressing the polypeptide or to purify the polypeptides by affinity chromatography.

Antibodies against polypeptides may also be employed to treat CVH, in particular for the treatment of IBS.

To determine the amount of protein, the antibodies are used in conventional immunological techniques. Suitable immunological techniques are well known to those skilled in the art and include for example, ELISA, Western Blot analysis, competitive or sandwich immunoassays and the like. As is otherwise well known they all depend on the formation of an antigen-antibody immune complex wherein for the purpose of the assay, the antibody can be detectably labeled with, e.g. radio-, enzyme or fluorescent labels or it can be immobilized on insoluble carriers.

For example in an ELISA screening format the antibody is added to a solid phase (for example the bottom of a microplate) which is coated with either the protein or a peptide fragment thereof coupled to a carrier (such as BSA), and then, adding an anti-immunoglobin antibody (for example when the immunization is performed in mice, an anti-mouse immunoglobulin antibody is used, e.g. sheep-anti-mouse immunoglobulin (Ig)) conjugated with a detectable label such as an enzyme, preferably horseradish peroxidase, or a radioactive isotope such as ¹²⁵I.
the individual nucleic acid and/or gene product can be initially used in a screening method to identify "candidate compounds" that bind specifically to the particular gene or gene product. Once identified, the candidate compound can be further used in cell-based or whole animal-based assays to determine its effect on expression of the particular nucleic acid, or expression or activity (*i.e.* function) of the gene product, relative to an untreated control cell or animal expressing the same nucleic acid and/or gene product. For the presentmethod , expression can also be detected in cells further treated or untreated with drugs commonly used to treat IBS, e.g. probiotics, including Lactobacillus and Bifidobacterium; anti-inflammatory agents, such as locally active 5-ASA compounds or corticosteroids, such as for example budenoside; mast cell stabilizers, PAR-2 antagonists and approaches that inhibit caspase activity, such as for example *N*¹-3-methylbutyryl-*N*⁴-6-aminohexanoyl-piperazine; CNI-1493 or pralnacasan. Cell culture assays using colon cells, e.g., Caco-2 or HT-29 cells, may be used to determine whether a test compound functions as a modulator of expression., For example cells are contacted with a test compound and the effect of the compound on the expression is evaluated relative to a corresponding cell not contacted with a test compound. As used herein, the term "corresponding cell" refers to a cell in a separate sample from that of the test sample that is preferably of the same cell-type from the same tissue-type as the cell being tested.
The presents disclosure provides a screening method to identify and obtaining a candidate compound for the treatment of CVH, in particular for the treatment of IBS, said method comprising;
a) incubating an IBS-MSG product with the compound to be tested; and
b) determining the capability of said compound to bind with the IBS-MSG product; wherein a compound capable of binding to the IBS-MSG product is a candidate compound for the treatment of IBS.
In these binding assays the IBS-MSG product typically consists of the polypeptide encoded by said gene or fragments thereof and the capability of the test compound to bind with said polypeptide is determined using art known procedures, such as for example described in Ausubel et al. (Current Protocols in Molecular Biology, Wiley Interscience, New York, 2001). In an alternative version the IBS-MSG product is a polynucleotide transcribed from the IBS-MSG gene or a fragment thereof.

In one particular working example, a candidate compound that binds to a polypeptide may be identified using a chromatography-based technique. For example, a recombinant polypeptide may be purified by standard techniques from cells engineered to express the polypeptide (e.g. those described above) and may be immobilized on a column. A solution of candidate compounds is then passed through the column, and a compound specific for the immobilized polypeptide is identified on the basis of its ability to bind to the polypeptide and be immobilized on the column. To isolate the compound, the column is washed to remove non-specifically bound molecules, and the compound of interest is then released from the column and collected. Similar methods may be used to isolate a compound bound to a polypeptide microarray. Compounds isolated by this method (or any other appropriate method) may, if desired, be further purified (e.g. by high performance liquid chromatography). In addition, these candidate compounds may be tested for their ability to alter (e.g. increase or decrease) the activity of a polypeptide. Compounds isolated by this approach may also be used, for example, as therapeutics to treat 1BS in a human subject. Compounds that are identified as binding to a polypeptide with an affinity constant less than or equal to 10 µM are considered particularly useful and are hereinafter also referred to as specific binding agents.

Alternatively, the binding assay further comprises the presence of a specific binding agent for the IBS-MSG of interest, i.e. either an antibody or another agent known to bind with the gene of interest. For the IBS-MSGs, a list of known commercially available antibodies and of known agonists is provided in the lists hereinafter. In the binding assay, the capability of the test compound to bind with the IBS-MSG is assessed by measuring the effect of the test compound on the interaction between the IBS-MSG and said specific binding agent.

### Examples of commercially available Antibodies (monoclonal or polyclonal) for genes listed in Table 1:

Anti-Human CASP1 Antibody
   (Abnova Corporation, Calbiochem, Novus Biologicals)
Anti-Human NCF4 Antibody
   (Abnova Corporation, Abcam, GeneTex, Novus Biologicals)
Anti-Human Lysozyme Antibody
   (BIODESIGN International)
Anti-Human PSME2 Antibody
   (Abnova Corporation, Novus Biologicals)
Anti-Human HELLS Antibody
   (Abnova Corporation, Bethyl Laboratories, GeneTex, Novus Biologicals)
Anti-Human COP1 Antibody
   (Abnova Corporation, IMGENEX, Novus Biologicals)
Anti-Human MCM5 Antibody
   (Abcam, AbD Serotec, BD Biosciences Pharmingen, Bethyl Laboratories,
   BioLegend, GeneTex, Lab Vision, Novus Biologicals, Spring Bioscience) Anti-Human TAP2 Antibody
   (Abgent, BD Biosciences Pharmingen)
Anti VSIG2 Antibody
   (Abcam: mouse monoclonal Cortical Thymocytes antibody, ab24235 - reacts with human)

### Examples of agonists for proteins encoded by the genes listed in Table 1:

LYSOZYME activation (agonists):
   - cyclosporin A (induction of lysozyme release)
   - 1-ethyl-benzimidazolinone (1-EBIO)
   - Carbachol
   - Thapsigargin
   - Phenylephrine
NADPH OXIDASE activation:
   - angiotensin II [Ang II]
   - PMA
   - TNF-alpha
   - growth factors
   - thrombin
   - phorbol myristate acetate (PMA)
PSME2 and TAP2 activation:
   - interferon-gamma

For detection of molecules capable of binding to the genes of interest using the aforementioned screening assays, the molecule that specifically binds to the gene of interest (*e.g.* antibody, agonist or polynucleotide probe) can be detectably labeled by virtue of containing an atom (*e.g*. radionuclide), molecule (*e.g*. fluorescein), or complex that, due to a physical or chemical property, indicates the presence of the molecule. A molecule may also be detectably labeled when it is covalently bound to or otherwise associated with a "reporter" molecule (*e.g*. a biomolecule such as an enzyme) that acts on a substrate to produce a detectable atom, molecule or other complex. Suitable detectable labels include any composition detectable by spectroscopic, photochemical, biochemical, immunochemical, electrical, optical or chemical means. Useful abels include biotin for staining with labeled avidin or streptavidin conjugate, magnetic beads (*e.g*. Dynabeads'), fluorescent dyes *(e.g.* fluorescein, fluorescein-isothiocyanate (FITC), Texas red, rhodamine, green fluorescent protein, enhanced green fluorescent protein, lissamine, phycoerythrin, Cy2, Cy3, Cy3.5, Cy5, Cy5.5, Cy7, FluorX [Amersham], SyBR Green I & II [Molecular Probes], and the like), radiolabels (*e.g*. ³H,¹²⁵I, ³⁵S, ¹⁴C, or ³²P), enzymes (*e.g*. hydrolases, particularly phosphatases such as alkaline phosphatase, esterases and glycosidases, or oxidoreductases, particularly peroxidases such as horse radish peroxidase, and the like), substrates, cofactors, inhibitors, chemilluminescent groups, chromogenic agents, and colorimetric labels such as colloidal gold or colored glass or plastic (*e.g*. polystyrene, polypropylene, latex, etc.) beads. Patents teaching the use of such labels include U. S. Pat. Nos. 3,817, 837; 3,850, 752; 3,939, 350; 3,996, 345; 4,277, 437; 4,275, 149; and 4,366, 241.

Means of detecting such labels are well known to those of skill in the art. Thus, for example, chemilluminescent and radioactive labels may be detected using photographic film or scintillation counters, and fluorescent markers may be detected using a photodetector to detect emitted light (*e.g*. as in fluorescence-activated cell sorting). Enzymatic labels are typically detected by providing the enzyme with a substrate and detecting a colored reaction product produced by the action of the enzyme on the substrate. Colorimetric labels are detected by simply visualizing the colored label. Thus, for example, where the label is a radioactive label, means for detection include a scintillation counter, photographic film as in autoradiography, or storage phosphor imaging. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Also, simple colorimetric labels may be detected by observing the color associated with the label. Fluorescence resonance energy transfer has been adapted to detect binding of unlabeled ligands, which may be useful on arrays.

Evaluation of binding interactions may further be performed using Biacore technology, wherein the IBS-MSG polypeptide or its binding partner is bound to a micro chip, either directly by chemical modification or tethered via antibody-epitope association (e. g. antibody to the IBS-MSG polypeptide), antibody directed to an epitope tag (e. g. His tagged) or fusion protein (e.g. GST). A second protein or proteins is/are then applied via flow over the"chip"and the change in signal is detected. Finally, test compounds are applied via flow over the"chip"and the change in signal is detected.

Classes of compounds that may be identified by such screening assays include, but are not limited to, small molecules (e. g. organic or inorganic molecules which are less than about 2 kd in molecular weight, more preferably less than about 1 kd in molecular weight, and/or are able to cross the blood-brain barrier or gain entry into an appropriate cell and affect the expression of the relevant gene or the activity of the relevant gene product). Compounds identified by these screening assays may also include polypeptides, such as soluble peptides, fusion peptides, members of combinatorial libraries (such as those described by Lam et al., Nature 1991,354 : 82-84; and by Houghten et al., Nature 1991,354 : 84-86); members of libraries derived by combinatorial chemistry, such as molecular libraries of D- and/or L-configuration amino acids; phosphopeptides, such as members of random or partially degenerate, directed phosphopeptide libraries (see,e. g., Songyang et al., Cell 1993,72 : 767-778); peptide libraries derived from the"phage method" (Scott and Smith, Science 1990,249 : 386-390; Cwirla, et al., Proc. Natl. Acad. Sci. USA 1990,87 : 6378- 6382; Devlin et al., Science 1990,49 : 404-406); chemicals from other chemical libraries (Geysen et al., Molecular Immunology 1986,23 : 709-715; Geysen et al., J. Immunologic Methods 1987,102 : 259-274; Fodor et al., Science 1991,251 : 767-773;. Furka etal., 14th International Congress of Biochemistry 1988, Volume & num;5, Abstract FR: 013; Furka, Int. J. Peptide Protein Res. 1991,37 : 487-493; U. S. Patent No. 4,631, 211; U. S. Patent No. 5,010, 175; Needels et al., Proc. Natl. Acad. Sci. USA 1993,90 : 10700-4; Ohlmeyer et al., Proc. Natl. Acad. Sci. USA 1993,90 : 10922-10926; PCT Publication No. WO 92/00252; and PCT Publication No. WO 94/28028); and large libraries of synthetic or natural compounds available from a variety of sources, including Maybridge Chemical Co. (Trevillet, Cornwall, UK), Comgenex (Princeton, NJ), Brandon Associates (Merrimack, NH), Microsource (New Milford, CT), Aldrich (Milwaukee, WI), Pan Laboratories (Bothell, WA), and MycoSearch (NC) (see, e. g. Blondelle et al., TIBTech 1996,14 : 60).

### Diagnostic Assays

Polynucleotides described herein an be used as diagnostic reagents. Detection of a mutated form of an IBS-MSG selected from the group consisting of IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4 and MUC20; in particular from the group consisting of IBS1 [SEQ ID NO: 15], COP1, PSME2, F13A1, NCF4, CSF1R, M160, KCNS3, VSIG2; more in particular from the group consisting of IBS1 [SEQ ID NO: 15], PSME2, F13A1, NCF4, CSFR1 AND VSIG2; even more in particular from the group consisting of MUC20, VSIG2 and VSIG4; most particular the IBS-MSG IBS1 [SEQ ID NO: 15], will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques.

It will thus be appreciated a method is provided of diagnosing a pathological condition or a susceptibility to a pathological condition in a subject related to CVH, in particular IBS, comprising:
(a) determining the presence or absence of a mutation in apolynucleotide according ; and
(b) diagnosing a pathological condition or susceptibility to a pathological condition based on the presence or absence of said mutation.

The methods further include methods of determining whether or not a sample is indicative of IBS and/or a particular stage of IBS and/or indicative of a susceptibility of IBS based on step (a) described above.

Nucleic acids for diagnosis may be obtained from a subject's cells, such as from blood (including total blood, serum, plasma and in particular white blood cells), urine, saliva, fecal sample, fecal cells, tissue biopsy (in particular colon biopsy) or autopsy material. The genomic DNA may be used directly for detection or may be amplified enzymatically by using PCR or other amplification techniques prior to analysis. mRNA or cDNA may also be used in similar fashion. Deletions and insertions can be detected by a change in size of the amplified product in comparison to the normal genotype. Point mutations can be identified by hybridizing amplified DNA to labeled mammalian purine permease nucleotide sequences. Perfectly matched sequences can be distinguished from mismatched duplexes by RNase digestion or by differences in melting temperatures. DNA sequence differences may also be detected by alterations in electrophoretic mobility of DNA fragments in capilary electrophoresis columns or gels, with or without denaturing agents, or by direct DNA sequencing (e.g., Myers et al., Science (1985)230:1242). Sequence changes at specific locations may also be revealed by specific restriction endonucleases, nuclease protection assays, such as RNase and S 1 protection or a chemical cleavage method (see Cotton et al., Proc Natl Acad Sci USA (1985) 85: 4397-4401). Alternatively, an array of oligonucleotides probes that specifically bind to the IBS-MSGs can be constructed to conduct efficient screening of e.g., genetic mutations. Array technology methods are well known and have general applicability and can be used to address a variety of questions in molecular genetics including gene expression, genetic linkage, and genetic variability (see for example: M.Chee et al., Science, Vol 274, pp 610-613 (1996)).

The diagnostic assays offer a process for diagnosing or determining a susceptibility to IBS through detection of mutations in the IBS-MSGs by the methods described. In addition, such disease may be diagnosed by methods comprising determining from a sample derived from a subject an abnormally decreased or increased level of polypeptide or mRNA, as well as by determining from said samples the presence of protein derivatives compared to the normal structure. Decreased or increased expression can be measured at the RNA level using any of the methods well known in the art for the quantitation of polynucleotides, such as, for example; nucleic acid amplification, for instance via PCR, RT-PCR; RNase protection; Northern blotting and other hybridization methods. Assay techniques that can be used to determine levels of a protein, such as a polypeptide , in a sample derived from a host are well-known to those of skill in the art. Such assay methods include radioimmunoassays, competitive-binding assays, Western Blot analysis and ELISA assays. Assay techniques that can be used to determine the presence of protein derivatives or variants comprise amongst others mass spectrometry.

Thus in another aspect, the present invention provides a method for detecting IBS in a subject, said method comprising:
(a) determining, in a biological sample of said subject, the level of gene transcription of an IBS-MSG;
(b) comparing the level of gene transcription with the level of gene transcription in a normal control sample; and
(c) producing a diagnosis based on the result from step b).
The methods further include methods of determining whether or not a sample is indicative of IBS and/or a particular stage of IBS and/or indicative of a susceptibility of IBS based on steps (a) and (b) described above.

The IBS-MSG as used in the diagnostic methods of the present invention is defined in the claims

In a particular embodiment, the biological sample of said patient is a sample of colon tissue, more particularly, a sample of mucosal colon tissue.

For each of the embodiments described thereof, step (b) consists of comparing the expression level of genes as mentioned in the claims with the level of transcription of said genes in a healthy control sample.

In any of the aforementioned methods for detecting and/or monitoring IBS in a subject, the use of the IBS-MSGs as identified in the present application may be combined with other genes.

In order to detect IBS in a subject one would have to compare the expression levels of the IBS-MSGs in a sample of said subject with a normal control sample. Changes in the expression levels of the IBS-MSGs in the sample of said subject compared to the expression levels of said genes in the control sample are indicative for a diagnosis of, or susceptibility to IBS in said subject. For example, if the level of any of the following IBS-MSGs: IBS1 [SEQ ID NO: 15], VSIG2 or MUC20 is increased (e.g. 30%, 40%, 50%, 60%, 70%, 80%, 90%, 100%, 110%, 120%, 130%, 140%, 150% or more), relative to the control sample, this is considered a positive indicator for IBS in said subject. In another example, if the level of any of the following IBS-MSGs: COP1, PSME2, F13A1, NCF4, CSF1R, M160, KCNS3, LYZ, MS4A4A, HELLS, RFC4, MCM5, TAP2, LRAP, DTL or VSIG4 is decreased (e.g. 30%, 40%, 50%, 60%, 70%, 80%, 90% or more), relative to the control sample, this is considered a positive indicator for IBS in said subject. Additionally or alternatively, the differences in expression can be expressed as 'fold-changes' compared to the expression level observed in control samples. Herein, a 1,4 fold change will correspond to an increase of 40%, etc. Generally, a decrease in expression will be referred to as 0.6 fold change for a decrease of 40%.

As described hereinbefore, the level of gene transcription is determined either at the protein level, preferably using antibodies that bind to the IBS-MSG polypeptide, or at the gene transcription level, preferably using probes that specifically bind to an oligonucleotide transcribed from said IBS-MSG, preferably at the cDNA or mRNA level. In a particular embodiment the level of gene transcription is determined using array technology, either at the oligonucleotide level using specific probes as described hereinbefore.

Hence, in a further embodiment the present invention, the level of gene expression in the aforementioned methods is assessed using a probe that specifically binds to cDNA or mRNA of the gene of interest; in particular using microarray technology.
It is accordingly an object of the present invention to provide a method for determining and monitoring IBS in a subject, wherein the level of gene transcription is assessed using an array of oligonucleotide probes that bind to the IBS-MSGs as claimed;. As already mentioned hereinbefore, the arrays of oligonucleotide probes for the IBS-MSGs are optionally combined with probes that specifically bind to other genes,.

Protein arrays are typically solid-phase, ligand binding assay systems using immobilized proteins on surfaces which include glass, membranes, microtiter wells, mass spectrometer plates and beads or other particles. Automated multi-well formats are the best developed and automated 96-well plate-based screening systems are the most widely used. For a description of protein arrays see US patents 6,475,809; 6,406,921 and 6,197,599; and PCT publications WO 00/04389 and WO 00/07024.

For construction of arrays, sources of proteins include cell-based expression systems for recombinant proteins, purification from natural sources, production in vitro by cell-free translation systems, and synthetic methods for peptides. For capture arrays and protein function analysis, it is important that proteins should be correctly folded and functional; this is not always the case, e. g. where recombinant proteins are extracted from bacteria under denaturing conditions, whereas other methods (isolation of natural proteins, cell free synthesis) generally retain functionality. However, arrays of denatured proteins are useful in screening antibodies for cross-reactivity, identifying auto-antibodies and selecting ligand binding proteins.

The immobilization method used should be reproducible, applicable to proteins of different properties (size, hydrophilic, hydrophobic), amenable to high throughput and automation, and compatible with retention of fully functional protein activity. Both covalent and noncovalent methods of protein immobilization are used. Substrates for covalent attachment include glass slides coated with amino-or aldehyde-containing silane reagents (Telechem). In theVersalinx' system (Prolinx), reversible covalent coupling is achieved by interaction between the protein derivatized with phenyldiboronic acid, and salicylhydroxamic acid immobilized on the support surface. Covalent coupling methods providing a stable linkage can be applied to a range of proteins. Noncovalent binding of unmodified protein occurs within porous structures such as HydroGel (PerkinElmer), based on a 3-dimensional polyacrylamide gel.

A method for identifying and/or monitoring IBS in a subject said method is disclosed comprising;
a) determining, in a biological sample of said subject, the protein level of at least one IBS-MSG protein;
(b) comparing the protein level with the protein level in a normal control sample; and
(c) producing a diagnosis based on the result from step b).

Preferably, the protein level is determined using at least one antibody that binds to an IBS-MSG protein. In particular using one or more antibodies, each of which binds to a polypeptide encoded by an IBS-MSG selected from IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4 and MUC20; in particular from the group consisting of IBS1 [SEQ ID NO: 15], COP1, PSME2, F13A1, NCF4, CSF1R, M160, KCNS3, VSIG2; more in particular from the group consisting of IBS1 [SEQ ID NO: 15], PSME2, F13A1, NCF4, CSFR1 AND VSIG2; even more in particular from the group consisting of MUC20, VSIG2 and VSIG4; In a specific example, the protein level is determined using an antibody specific for IBS1 [SEQ ID NO: 15] .

In an alternative version the method is not limited to at least one protein , but requires the simultaneous assessment of the expression levels of the group of proteins identified as being involved in IBS. The simultaneous assessment of the expression levels of the group of proteins can be done using array technology, in particular using immunological methods (such as ELISAs and RIAs). As mentioned hereinbefore, in protein arrays the primary agent, typically an antibody or protein that recognizes the IBS proteins is bound to a solid support (e.g. a membrane or a microtiter plate). Using this solid support the IBS proteins can be extracted from the biological sample and quantified using a secondary agent (e.g. a second antibody recognizing a second epitope in the IBS protein or an antibody or protein that recognizes the primary antibody) conjugated with a detectable label such as an enzyme, preferably horseradisch peroxidase, or a reactive isotope such as ¹²⁵I.

Methods are provided for detecting and/or monitoring IBS in a subject, said method comprising;
a) contacting a biological sample of said subject with at least one agent that specifically binds with an IBS-MSG polypeptide;
b) determining the level of binding of the agent to the polypeptide;
c) comparing the level of binding of the agent in said biological sample with the level of binding of the agent in a normal control sample; and
d) producing a diagnosis or determining whether or not the sample is indicative of IBS and/or a particular status of IBS based on the result of step c).

As already mentioned hereinbefore, an assay is disclosed using a protein array of IBS-MSG polypeptide specific antibodies; in one example the two or more specific antibodies are each reactive with polypeptides of IBS-MSGs selected from the group consisting of IBS1 [SEQ ID NO: 15], COP1, PSME2, F13A1, NCF4, CSFR1, M160, KCNS3, LYZ, MS4A4A, HELLS, FRC4, MCM5, TAP2, LRAP, DTL, VSIG2, VSIG4 and MUC20; in particular reactive with polypeptides of IBS-MSGs selected from the group consisting of IBS 1 [SEQ ID NO: 15], COP1, PSME2, F13A1, NCF4, CSF1R, M160, KCNS3 and VSIG2; more in particular reactive with polypeptides of IBS-MSGs selected from the group consisting of IBS1 [SEQ ID NO: 15], PSME2, F13A1, NCF4, CSFR1 and VSIG2; even more in particular reactive with IBS-MSGs selected from the group consisting of MUC20, VSIG2 and VSIG4. As already mentioned hereinbefore, the protein arrays for the IBS-MSGs are optionally combined with agents that specifically bind to other genes, in particular selected from the group consisting of CASP1, FCGR2A and CKB.

In order to detect IBS in a subject one would have to compare the level of binding of the agent to the IBS-MSG polypeptides in a sample of said subject with the level of binding in a normal control sample. Changes in the binding levels are indicative for a diagnosis of, or susceptibility to IBS in said subject. For example, if the binding level of any of the following IBS-MSG polypeptides: IBS1 [SEQ ID NO: 15], VSIG2 or MUC20 is increased (e.g. 30%, 40%, 50%, 60%, 70%, 80%, 90%, 100%, 110%, 120%, 130%, 140%, 150% or more), relative to the control sample, this is considered a positive indicator for IBS in said subject. In another example, if the binding level of any of the following IBS-MSG polypeptides: COP1, PSME2, F13A1, NCF4, CSF1R, M160, KCNS3, LYZ, MS4A4A, HELLS, RFC4, MCM5, TAP2, LRAP, DTL or VSIG4 is decreased (e.g. 30%, 40%, 50%, 60%, 70%, 80%, 90% or more), relative to the control sample, this is considered a positive indicator for IBS in said subject

The diagnostic methods described herein can also be used to monitor the IBS in a subject or to determine the dosages of therapeutic compounds. In one example, a therapeutic compound is administered and the level of expression of an IBS-MSG is determined during the course of therapy.

Therapeutics that modulate the expression of any one or more of the IBS-MSGs are considered particularly useful. In one example, a therapeutic agent that decreases, by 10%, 20%, 30%, 40%, 50%, 60%, 70%, 80%, 90%, 100%, 110%, 120%, 130%, 140%, 150% or more, the expression level of any of the following IBS-MSGs: IBS 1 [SEQ ID NO: 15], VSIG2 or MUC20 during the course of therapy, is considered to be an effective therapeutic agent or an effective dosage of a therapeutic agent. In another example, a therapeutic agent that increases, by 10%, 20%, 30%, 40%, 50%, 60%, 70%, 80%, 90% or more the expression level of any of the following IBS-MSGs: COP1, PSME2, F13A1, NCF4, CSF1R, M160, KCNS3, LYZ, MS4A4A, HELLS, RFC4, MCM5, TAP2, LRAP, DTL or VSIG4 during the course of therapy, is considered to be an effective therapeutic agent or an effective dosage of a therapeutic agent.

### Diagnostic Kits

The invention also encompases kits for detecting the presence of an IBS-MSG product in a biological sample, the kit comprising the components required to carry claimed diagnostic assays and instructions for the use of the components for assessing expression of the IBS-MSGs in a biological sample and diagnosing IBS in a subject.

It is accordingly an object of the present invention to provide a diagnostic kit as claimed:
Diagnostic kits generally include a label or instructions for the intended use of the kit components and a reference sample to be used to establish a standard curve. In one example, the kit contains instructions for the use of the kit for the diagnosis of IBS. In yet another example, the kit contains instructions for the use of the kit to monitor therapeutic treatment or dosage regimens for the treatment of IBS. It will be understood that the reference sample values will depend on the intended use of the kit. For example, the sample can be compared to a normal reference value, wherein an alteration in the levels of one or more of the polypeptides or a metric using levels of one or more of the polypeptides is indicative of IBS, or a predisposition to IBS. In another example, a kit used for therapeutic monitoring can have a reference value that is indicative of IBS, wherein an alteration in the level of one or more of the polypeptides or a metric using levels of one or more of the polypeptides relative to the reference sample can be used to indicate therapeutic efficacy or effective dosages of therapeutic compounds.

### Therapeutic Utility

The present disclosure features methods and compositions for treating or preventing CVH, in particular for treating or preventing IBS in a subject. It has been discovered that levels of IBS 1 [SEQ ID NO: 15], VSIG2 and MUC20 are increased in subjects having IBS. Therefore, this includes methods and agents that decrease the expression levels or biological activity of any one or more of these polypeptides or nucleic acid molecules. Such agents which are described in more detail below, include compounds that down-regulate or inhibit the biological activity of any one or more of the above polypeptides; immunological/vaccine formulations; a purified antibody or antigen-binding fragment that specifically binds any one of the above polypeptides; antisense nucleobase oligomers; and dsRNAs targeting any of the above polypeptides.

In a first aspect, reduction of the biological activiy of the polypeptides that are upregulated in IBS will be established using a pharmaceutical composition comprising a therapeutically effective amount of an antagonist, e.g. peptide or small molecule compound, in combination with a pharmaceutically acceptable carrier or excipient.

This invention will be better understood by reference to the Experimental Details that follow, but those skilled in the art will readily appreciate that these are only illustrative of the invention as described more fully in the claims that follow thereafter. Additionally, throughout this application, various publications are cited. The disclosure of these publications is hereby incorporated by reference into this application to describe more fully the state of the art to which this invention pertains.

### EXPERIMENTAL PROCEDURES

To further understand the molecular mechanisms in the pathogenesis of IBS, microarray expression profiling study of mucosa of the sigmoid colon was performed using biopsies that were collected in the same manner as in routine clinical practice.

As explained in more detail hereinafter, our study included 36 IBS patients (21 IBS-D and 15 IBS-C) and 25 healthy control subjects. All patients fulfilled the Rome II criteria for IBS diagnosis (Thompson et al., 1999) and underwent a thorough clinical examination to exclude other gastrointestinal disorders. Patients were selected based on their predominant bowel dysfunction which was confirmed at the time of the study by means of a standard questionnaire. Two sigmoid colon biopsies were collected from each participant during a sigmoidoscopy examination. An additional third colon biopsy was collected 2-3 months later from 10 subjects (5 IBS patients and 5 healthy controls) in order to assess the stability of the molecular signatures in health and IBS.

### METHODS

### Recruitment of subjects and collection of colon biopsy samples

IBS participants were selected from an administrative database of 752 patients with IBS residing within 150 miles radius of Rochester (Minnesota, U.S.A.), and were recruited by mailing. All IBS patients had already been evaluated by a staff gastroenterologist by clinically indicated tests including endoscopy, biopsies and tests of rectal evacuation. Patients were selected based on their predominant bowel dysfunction which was confirmed at the time of study by means of a standard questionnaire (Talley et al., 1990). Healthy volunteers were recruited by public advertisement in Rochester, MN. All participants who responded to a letter inviting their participation signed informed consent for the study, which was approved by the Mayo Clinic Institutional Review Board. Every participant completed a validated bowel disease questionnaire (Talley et al., 1990) including questions to correspond to Rome II criteria (Thompson et al., 1999). The bowel disease questionnaire also includes a psychosomatic symptom checklist intended to identify somatization disorders and symptoms to characterize non-ulcer dyspepsia, and has been used extensively in epidemiological studies.

Participants attended the General Clinical Research Center in Charlton 7 to undergo a flexible sigmoidoscopy for the collection of colonic biopsies. Due to the risk of bleeding from the biopsy procedure, participants taking aspirin or anticoagulants were excluded if these medications could not be stopped at least 1 week prior to the endoscopy. Two phosphosoda enemas (Fleet@ enema, C.B Fleet, Lynchburg, VA) were administered one hour prior to sigmoidoscopy. Biopsies were taken from normal appearing mucosa only, i.e., areas with edema due to endoscope pressure were avoided. The sigmoidoscopy was performed without sedation as is the clinical standard at Mayo Clinic, and the subjects were monitored for 60 minutes after the biopsies to ensure that they are stable without signs of any bleeding or other complications. Using standard large size biopsy forceps, two sigmoid colon mucosal biopsies were collected from 15 IBS-C, 21 IBS-D patients and 25 healthy controls. Ten of these subjects (5 controls and 5 IBS patients) were randomly selected to have a second, IRB-approved sigmoidoscopy 2-3 months after the first collection in order to assess data reproducibility.

### Array processing and pre-processing of the data

Upon collection, colon biopsy samples were immediately submerged in 5 volumes of RNAlater solution (Ambion, Austin, TX) and stored at -20°C until further analysed. Tissue was homogenized in a mixer mill 501 (Qiagen, Venlo, The Netherlands) in RLT cell lysis buffer (Qiagen), followed by RNA extraction from the disrupted cells using the RNeasy mini kit (Qiagen) with DNase treatment on the column. One µg of total RNA was biotin labelled and hybridized on Human Genome U133 Plus 2.0 GeneChip microarrays according to the instructions of the provider (Affymetrix, Santa Clara, CA). Given the large number of samples (n=132), this processing in the laboratory was performed in 4 different batches, each comprising samples from both IBS and healthy subjects. Gene expression summary values for raw Affymetrix GeneChip data were computed using the gcRMA algorithm (Wu et al. 2004), which does background adjustment, quantile normalization and summarization, taken GC affinities into account. PANP (Warren et al., 2006) was used for calling the detection of genes absent or present, and filtered genes when they were called present in at least 50% of the samples in one treatment group (McClintick and Edenberg, 2006). An effect of the different sample processing batches remained apparent after normalization. This technical source of variation was corrected for by modelling the expression levels in function of batch of origin in a one-way ANOVA, and using the residuals of this model for all subsequent analyses. Finally, to avoid misleading results due to pseudoreplication (Hurlbert 1984), the expression values of the replicated samples were averaged per patient for the SAM and PAM analyses (see below).

### Assessing concordance of repeated measurements

To quantify the sample reproducibility over time and tissue space for the same patient, concordance coefficients (Lin 1989) were calculated for the 1000 most variable gene probes, as well as for the set of 32 gene probes that were found to be predictive for IBS disease status in the PAM analysis.

### Testing for differentially expressed genes

Significance analysis of microarrays (SAM) was applied to identify differentially expressed genes in IBS-diseased versus healthy persons, using a D of 0.05 (Tusher et al., 2001). An alternative, more rigorous statistical model was also applied on the raw data, (i.e. preprocessed data of all biopsy samples before batch correction), by application of mixed ANOVA with batch and disease status as fixed and patient as a random effect, and with FDR correction (Storey et al., 2003).

### Classification

For disease status prediction, Predictive Analysis of Microarrays (PAM) was applied, which is an enhanced variant of nearest centroid classification using shrunken centroids (Tibshirani et al., 2002). Samples from 8 IBS patients and 8 healthy subjects were kept independent from the model building step to assess the model's predictive power so as to check for possible overfitting.

### Hierarchical clustering

To identify an underlying structure in the molecular signatures, hierarchical clustering (Spotfire DecisionSite 8.2 software) was applied on a set of 16 gene probes that were selected both in the PAM and SAM analysis.

### URLs

SAM software is available at http://www-stat.stanford.edu/∼tibs/SAM/.
PANP software is available at http://people.brandeis.edu/∼dtaylor/PANP/.

### RESULTS & DISCUSSION

### Sample analysis

Biotin-labelled total RNA prepared from each of the colon biopsy samples was hybridised on Human Genome U133 Plus 2.0 GeneChip microarrays (Affymetrix). Pre-processing of the generated raw data files, including background adjustment, data normalization and transformation, and correction for technical batch variation, revealed profiles of gene expression summary values for each sample that were used in all further analyses.

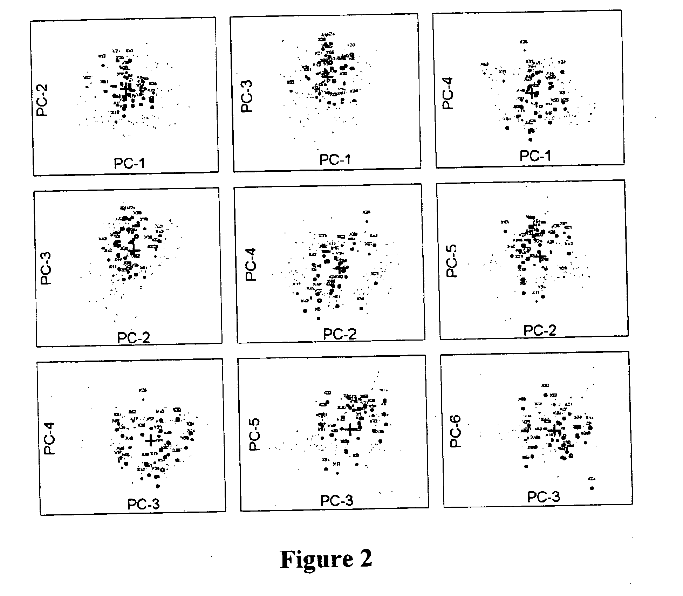

A prerequisite for any useful biomarker is the reproducibility of the gene expression profiles within individual subjects. The concordance coefficient, which measures how well a set of points matches the identity line (Lin 1989), was measured between repeated samples of the same patient using the 1,000 most variable gene probes on the microarray. Both the concordance between two simultaneously collected samples (0.7 ± 0.03), as well as between two samples collected from the same person with an interval period of 3 months (0.41 ± 0.03) significantly exceeded the overall concordance (0.25 ±0.12; Mann-Whitney U test; respectively W = 3510, p<0.0001 and W = 6744, p<0.0001). No significant differences in the concordance values were observed between IBS patients and healthy controls (Figure 1A). Since the overall expression profiles of sigmoid colon biopsies were relatively stable for two site and two time sample collections, the gene probe expression levels of the two collected colon samples per patient was averaged for the subsequent analyses like significance analysis of microarray (SAM) and classification (see below). Moreover, the selection of the subgroup for repeat sample collection was representative of the original study group. This was assessed by randomly selecting 10 individuals for the repeat sample collection: 5 healthy controls and 5 IBS patients, without considering of any selection criteria. A posteriori, it was verified whether this subgroup of subjects was representative for the whole cohort using spectral map analysis. The graphical output of this analysis - summarizing the combined effect of the six first principal components - shows that the subjects selected for repeat sampling are indeed representative for the whole cohort (Figure 2).

Next, a search was performed for differentially-expressed genes between IBS patients and healthy controls, using the SAM algorithm (Tusher et al., 2001). At a 5% false discovery rate, 25 gene probe sets were found to be differentially expressed between IBS and healthy persons at a significance level of 0.1 for the q-values. These 25 significant (q<0.1) gene probe sets represented 20 different genes: 4 up-regulated and 16-down regulated in IBS patients compared to healthy controls (Table 1). An alternative statistical approach was also applied on the normalized raw data. This was a mixed ANOVA model with batch and disease status as fixed and patient as random effect, and with false discovery rate correction (Storey et al., 2003). This analysis resulted in a very similar list of genes with q-values comparable to the SAM analysis (Table 1). The genes that were significantly differentially expressed reflected subtle changes in expression levels: only a few of the significant genes had changes in expression level > 1.5-fold between IBS patients and healthy controls.

**Table 1**

| **Probe set** | **Gene symbol** | **q-value SAM** | **q-value ANOVA** | **Fold change** | **Gene annotation** |
|---|---|---|---|---|---|
| **HIGHER expression in IBS patients versus controls** | | | | | |
| 225809_at | *IBS1* | **0.018** | **0.03** | 1.41 | DKFZP564O0823 (IBS1) |
| 204687_at | *IBS1* | **0.018** | **0.01** | 1.24 | DKFZP564O0823 (IBS1) |
| 226622_at | *MUC20* | **0.018** | **0.05** | 1.52 | Mucin 20 |
| 229369_at | *VSIG2* | **0.023** | **0.01** | 1.20 | V-set and immunoglobulin domain containing, 2 |
| 231941_s_at | *MUC20* | **0.030** | 0.07 | 1.47 | Mucin 20 |
| 200884_at | *CKB* | **0.039** | **0.05** | 1.29 | Creatine kinase, brain |

| **LOWER expression in IBS patients versus controls** | | | | | |
|---|---|---|---|---|---|
| 223655_at | *M160* | **0.018** | **0.05** | 0.69 | Scavenger receptor cysteine-rich type 1 protein M160 (CD 163 antigen-like 1) |
| 204787_at | *VSIG4* | **0.023** | **0.05** | 0.66 | V-set and immunoglobulin domain containing, 4 |
| 211368_s_at | *CASP1* | **0.030** | **0.05** | 0.75 | Caspase 1, apoptosis-related cysteine peptidase (interleukin 1, beta, convertase) |
| 205147_x_at | *NCF4* | **0.030** | **0.05** | 0.70 | Neutrophil cytosolic factor 4, 40kDa |
| 1555745_a_at | *LYZ* | **0.030** | 0.11 | 0.48 | Lysozyme |
| 205968_at | *KCNS3* | **0.039** | 0.10 | 0.66 | Potassium voltage-gated channel, delayed-rectifier, subfamily S, member 3 |
| 211367_s_at | *CASP1* | **0.039** | 0.06 | 0.75 | Caspase 1, apoptosis-related cysteine peptidase (interleukin 1, beta, convertase) |
| 201762_s_at | *PSME2* | **0.045** | **0.01** | 0.84 | Proteasome activator subunit 2 (PA28 beta) |
| 211366_x_at | *CASP1* | **0.049** | 0.06 | 0.76 | Caspase 1, apoptosis-related cysteine peptidase (interleukin |
| 219607_s_at | *MS4A4A* | 0.056 | 0.13 | 0.74 | 1, beta, convertase) Membrane-spanning 4-domains, subfamily A, member 4 |
| 220085_at | *HELLS* | 0.058 | 0.07 | 0.82 | Helicase, lymphoid-specific |
| 1552703_s_a | *COP1* | 0.080 | 0.06 | 0.81 | Caspase 1 dominant-negative inhibitor pseudo-ICE |
| 203561_at | *FCGR2A* | 0.080 | 0.08 | 0.77 | Fc fragment of IgG, low affinity IIa, receptor (CD32) |
| 204023_at | *RFC4* | 0.084 | 0.06 | 0.83 | Replication factor C (activator 1) 4, 37kDa |
| 206011_at | *CASP1* | 0.084 | 0.11 | 0.77 | Caspase 1, apoptosis-related cysteine peptidase (interleukin 1, beta, convertase) |
| 216237_s_at | *MCM5* | 0.084 | 0.08 | 0.76 | MCM5 minichromosome maintenance deficient 5, cell division cycle 46 (S. cerevisiae) |
| 225973_at | *TAP2* | 0.084 | 0.16 | 0.71 | Transporter 2, ATP-binding cassette, subfamily B (MDR/TAP) |
| 219759_at | *LRAP* | 0.084 | 0.21 | 0.39 | Leukocyte-derived arginine aminopeptidase |
| 218585_s_at | *DTL* | 0.084 | 0.11 | 0.79 | Denticleless homolog (Drosophila) |

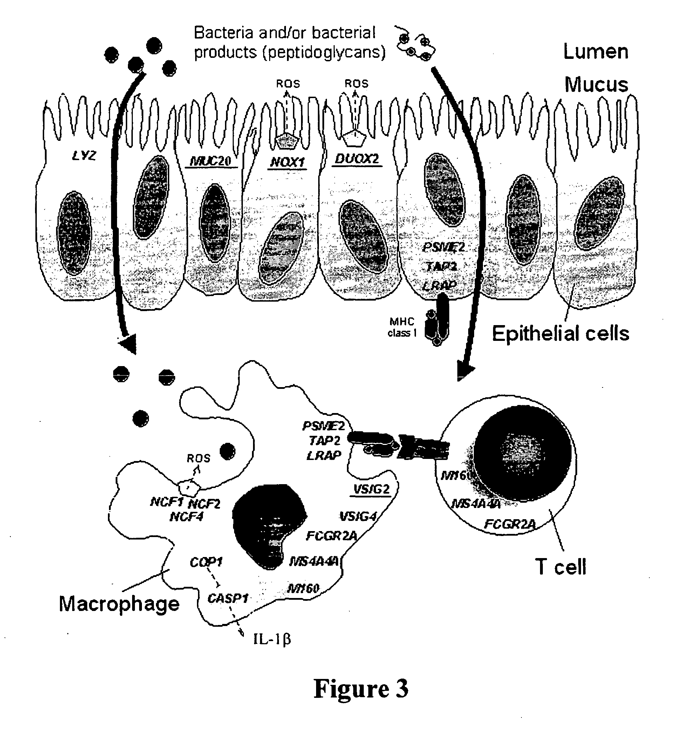

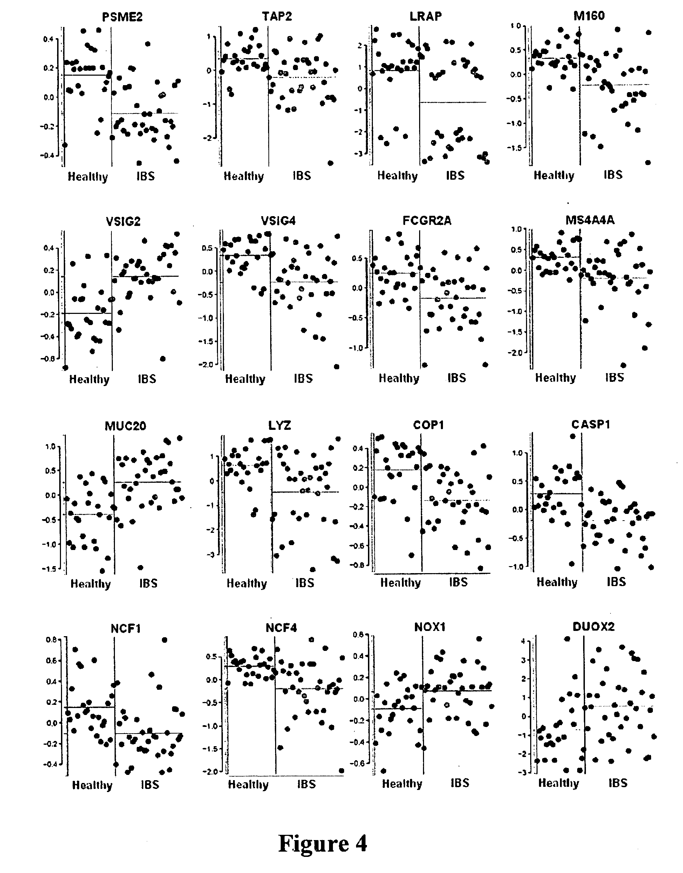

It is remarkable to note that the majority of the 20 identified significant genes play a role in the immune response or the host defense system against microbial invasion. A schematic overview of the differentially expressed genes in mucosal colon of IBS patients versus healthy controls is provided in Figure 3. Plots of the expression levels of some of the significant genes with differential expression between IBS diseased and healthy persons are shown in Figure 4.

### Alterations in genes affecting antigen processing

At least three genes with significantly lower expression levels in the colonic mucosa of IBS patients play an essential role in the pathway of antigen processing and presentation by the major histocompatibility I complex: *PSME2* (proteasome activator subunit 2, PA28 beta), *TAP2* (transporter 2, ATP-binding cassette, subfamily B), and LRAP (leukocyte-derived arginine aminopeptidase). PA28 is essential in the assembly of the cytosolic immunoproteasome complex, that is responsible for antigen processing of class I major histocompatibility complex (MHC) peptides (Preckel et al., 1999). TAP2 forms, together with TAP1, a heterodimeric transmembrane ATP-binding-cassette (ABC) transporter in the endoplasmic reticulum (ER) membrane that is essential for the delivery of antigenic peptides from the cytosol into the ER, where these peptides are loaded onto MHC class I molecules. TAP2, unlike TAP1, is very unstable in isolation, and the biogenesis of functional TAP depends on the assembly of pre-existing TAP1 with newly synthesized TAP2 but not vice versa, suggesting that mainly *TAP2* expression regulates the number of active transporter molecules (Keusekotten et al., 2006). In the ER, MHC class I molecules rely on aminopeptidases to trim precursors to antigenic peptides. LRAP, also named ER aminopeptidase 2 (ERAP2), is one of the key enzymes responsible for the hydrolysis of N-terminal amino acids of proteins or peptide substrates (Saveanu et al., 2005). Together, the significantly lower expression levels of *PSME2*, *TAP2*, and *LRAP* in mucosa of the colon of IB5 patients strongly suggest that the functional activity in MHC class I antigen presentation is modulated in these patients.

### Alterations in genes controlling immune response

In addition, 6 other significantly altered genes in our study are implicated in the immune response: *VSIG2*, *VSIG4*, *FCGR2A*, *MS4A4A*, *M160*, and *MUC20* (see Table 1 for respective q-values). The expression of *VSIG4*, a member of the family of V-set and immunoglobulin domain containing proteins (VSIG), is decreased, while the expression of another closely related family member, *VSIG2*, is higher in the mucosa of the colon of these subjects. The significance of the simultaneous but opposite alteration in gene expression of *VSIG4* and *VSIG2* in IBS patients is highly interesting given the recent discoveries on the function of these genes. The functional role of all VSIG family members has not yet been well studied, but VSIG4 appears to be critical in the regulation of an immune response mediated by phagocytosis and /or antigen presentation (Kim et al., 2005). Another significant gene alteration provides additional evidence for a modulated immune response system in the colon of IBS patients is *FCGR2A* (CD32), which encodes the immunoglobulin Fc receptor. These receptors are essential in the protection of the organism against foreign antigens by removing antigen-antibody complexes from the circulation. Fc receptors are present on monocytes, macrophages, neutrophils, natural killer (NK) cells, and T and B lymphocytes, and they participate in phagocytosis of immune complexes and modulation of antibody production by B cells (Unkeless JC, 1989). The expression of *MS4A4A* and CD163 molecule-like 1 (*M160*) are also lower in IBS patients. *MS4A4A* is at subunit homolog of another immunoglobulin receptor, and CD163 molecule-like 1 (*M160*) is a membrane-anchored member of the scavenger receptor cysteine-rich superfamily that is mainly expressed in cells associated with the immune system.

### Alterations in genes involved in local defense mechanisms

The expression of the cell surface associated mucin 20 protein (encoded by *MUC20),* on the other hand, is elevated in IBS patients. This gene is known to be predominantly expressed in the kidney, and an elevated expression has been described in epithelial cells from the proximal renal tubules in IgA nephropathy patients as well as several renal injury models (Higuchi et al., 2004). Moreover, stimulation of a renal tubular epithelial cell line with proinflammatory substances such as lipopolysaccharide (LPS), phorbol 12-myristate 13-acetate (PMA), or tumor necrosis factor alpha significantly increases *MUC20* mRNA expression. The elevated *MUC20* expression found in the colonic mucosa of IBS patients might reflect a response to a local injury or inflammation.

### Alterations in genes involved in host defence response to pathogens

At least 4 of the differentially expressed genes (*LYZ*, *CARP1*, *COP1*, *NCF4*) (Table 1) are involved in the host defense response to pathogens in the colon. The expression level of the anti-microbial agent lysozyme (*LYZ*), whose natural substrate is the bacterial cell wall peptidoglycan, is significantly lower in IB5 patients, suggesting that there may be the biological basis for a compromised innate immunity in the colon of IBS patients; this function requires further study. Paneth cells are secretory epithelial cells of the small intestinal mucosa, and a major source of anti-microbial peptides including lysozyme. These antimicrobial defenses may also be recruited to sites of inflammation in the colon through metaplasia of Paneth cells, most likely as a mucosal innate immunity response mechanism (Wehkamp et al., 2006). Whether the mucosal innate immune system in the colon is affected in IBS patients will require further study. Pro-inflammatory cytokines, such as interleukin-1β (IL- 1β) and gamma interferon (IFN-γ), are important components of the antimicrobial defense system. IL-18 is an IFN-γ-stimulating factor, and plays an important role in defense against a variety of gram-positive and -negative bacterial pathogens. The synthesis of IL-1β and IL-18 depends upon the proteolytic cleavage of their precursor proteins (pro- IL-1β and pro-IL-18) by the cysteine protease caspase 1 (CASP1), also known as interleukin-1β (IL-1β) converting enzyme. Caspase-1 deficient mice indeed have a major defect in IL-18 and IL-1β production in vivo, and this is accompanied by a resistance to lethal doses of endotoxin / LPS (Li et al., 1995). These mice are also two- to threefold more susceptible to lethal *Escherichia coli* infection than wild-type mice due to a failure of the innate host defense mechanism (Joshi et al., 2002). Caspase-1 dominant negative inhibitor (COP1), also known as pseudo-ICE, interacts physically with caspase-1 to block its activation, and hence, the secretion of IL- 1β and IL-18.

The expression of both CASP1 and COP1 is decreased in the mucosal colon of IBS patients. The genes for CASP1 and COP1 are contiguous on chromosome 11q, and it has been suggested that both genes are under similar transcriptional regulation, based on their identical tissue distribution (Druilhe et al., 2001). The effects of the altered mRNA expression of CASP1 and COP1 on the protein expression level and subsequently on the production of IL-1β and IL-18 are, however, unknown. In the samples of our study population, IL-10 was not differentially expressed, but an increased rectal mucosal mRNA expression of IL-1β has been reported recently in acquired post-infectious IBS patients (Gwee et al., 2003).

The NADPH oxidase complex was originally identified and characterized in phagocytes, where it plays an essential role in non-specific host defense against microbial organisms, by catalysing the generation of an oxidative burst of superoxide from oxygen and NADPH. The structure of NADPH oxidase consists of 2 membrane-bound elements (gp91^{phox} and p22^{phox}, encoded by *CYBB* and *CYBA,* respectively), three cytosolic components (p67^{phox}, p47^{phox} and p40^{phox}, encoded by *NCF2*, *NCF1*, and *NCF4*), and a low molecular weight G protein (either rac 1 or rac2, encoded by *RAC1* and *RAC2*). Activation of the enzyme complex is associated with the migration of the cytosolic components to the cell membrane, so that the complete oxidase can be assembled (DeCoursey and Ligeti, 2005). The lower expression of *NCF1* and *NCF4* in the mucosal colon of IBS patients may thus cause a shortage of cytosolic components transported to the membrane-bound elements of the NADPH oxidase enzyme complex, leading to a diminished activity of phagocyte-expressed NADPH oxidase. The essential catalytic core of the oxidase, gp91^{phox} (nowadays also called Nox2), belongs to a family of several very similar oxidases. Homologues of the NADPH oxidase complex were identified in numerous non-phagocytic cell types, including the Nox1 enzyme complex that is predominantly expressed in surface mucous epithelial cells of the colon (Kikuchi et al., 2000). The different enzyme complex homologues can be distinguished at the molecular level based on their -subunits composition. Compared to the Nox2 complex that is expressed in phagocytes, the Nox¹ complex shares the p22^{phox} (CYBA) subunit, but the gp91^{phox} *(CYBB),* p47^{phox} (*NCF1*), and p40^{phox} *(NCF4)* subunits are exchanged for *NOX1*, *NOXO1*, and *NOXA1*, respectively. Interestingly, a borderline elevated NOX1 expression in the colonic mucosa of at least a subset of IBS patients was found for several *NOX1* probe sets on the microarray (206418_at; 207217_s_at; 210808_s_at). It has been demonstrated that human colonic epithelial cells induce Nox1 expression and up-regulate superoxide production in response to IFN-γ (Kuwano et al., 2006) and to flagellin from Salmonella enteritidis (Kawahara et al., 2004). *Helicobacter pylori* lipopolysaccharide, known to cause a persistent inflammation and enhanced Tₕ1 immune response in human gastric mucosa, also stimulates the mRNA expression of *NOX1* and *NOXO1* in guinea pig gastric mucosal cells, followed by an upregulation of superoxide generation (Kusumoto et al., 2005). Another member of the family of NOX/DUOX oxidase genes is dual oxidase 2 (*DUOX2*), which is expressed all along the digestive tract, with the highest levels found in the epithelial cells of mucosal surfaces of caecum and sigmoid colon (El Hassani et al., 2005). *DUOX2* (but not *DUOX1*) mRNA expression levels were also found to be highly increased in the sigmoid colon mucosal biopsies of IBS patients (219727_at, q=0.15, 3.8-fold increase, which is the largest fold-change of all genes). This finding might be somewhat surprising, as DUOX2 is thought of as an inducible rather than a constitutively expressed dual oxidase (in contrast to DUOX1), and because DUOX2 generates a robust, self-limited response during infection or inflammation (Harper et al., 2005). Dual oxidases contain both an NADPH oxidase domain, responsible for H₂O₂ production, as well as a heme peroxidase domain that is closely related to several peroxidases including myeloperoxidase and lactoperoxidase. These oxidases provide an epithelial source of reactive oxygen species (ROS) such as superoxide and H₂O₂, which have an essential role in host defense mechanisms (Geiszt et al., 2003; El Hassani et al., 2005). Pro-inflammatory stimuli such as IFN-γ induce the expression of *DUOX2*, and lead to elevated H₂O₂ production (Harper et al., 2005). A direct role for dual oxidase in gut immunity was demonstrated in *Drosophila* : adult flies in which dual oxidase expression was silenced showed a marked increase in mortality even after a minor infection through ingestion of microbe-contaminated food. This effect could be reversed by the reintroduction of dual oxidase, demonstrating that the enzyme generates a unique epithelial oxidative burst that limits microbial proliferation in the gut (Ha et al., 2005). It is considered that the elevated DUOX2 expression is indicative for the existence of a mild but chronic inflammatory condition in the colon of IBS patients. Together, the altered expression of several members of multi-subunit NADPH oxidase / dual oxidase enzyme complexes in the colon of IBS patients provide further support for the hypothesis that the host defense response to bacteria and other invaders in the colon is disturbed in IBS patients.

### Upregulation of a novel gene in Irritable Bowel Syndrome

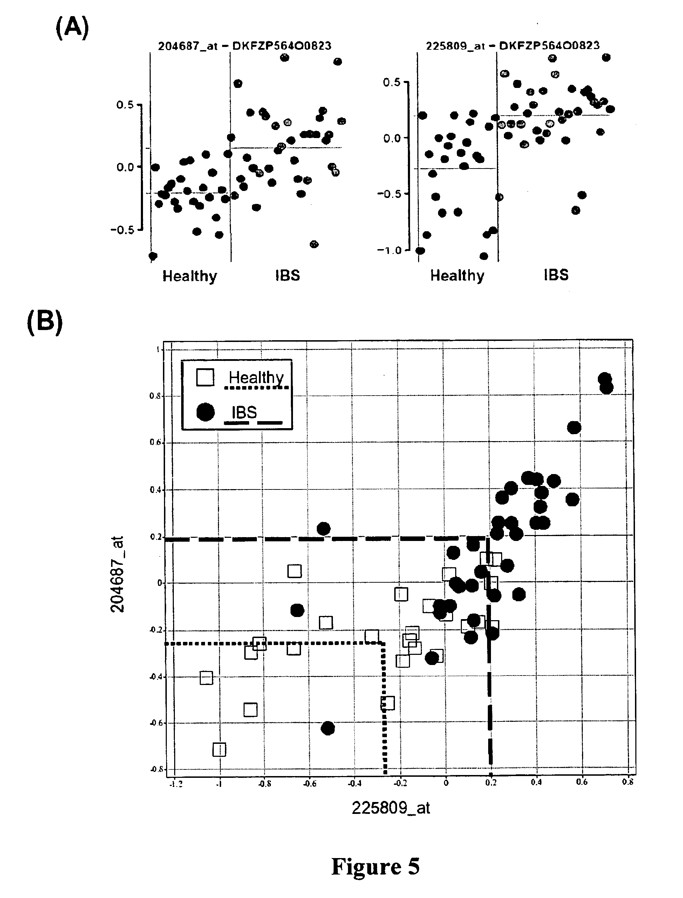

Finally, two of the most significantly up regulated probe sets in colon mucosal biopsies of IBS patients (Figure 5A) represent the same gene that is annotated in the public sequence databases as **DKFZP564O0823** [SEQ ID NO: 15] (NCBI GeneID: 25849). This gene is renamed ***IBS1*** (Irritable Bowel Syndrome 1) herein. *In silico* analysis demonstrates that the 5 kb cDNA sequence of *IBS1* contains an open reading frame of 930 bp that encodes a predicted plasma membrane protein of 310 amino acids (AA), including a signal peptide (20 AA), an extracellular region (238 AA), a transmembrane region (21 AA), and an intracellular region (31 AA).

A mouse homologue for the *IBS1* gene is known as RIKEN cDNA 9130213B05, and has been further annotated as a cell surface glycoprotein precursor. In rat, the *IBS1* homologue was identified as an up regulated gene in ventral prostate upon castration, associated with apoptosis, and was therefore annotated as Cipar-1 (castration induced prostatic apoptosis-related protein 1) or PARM-1 (prostatic androgen-repressed message 1) (Bruyninx et al., Endocrinology, 1999, 140:4789-4799; Comet et al., Prostate, 2003, 56:220-230).

Although no literature on the human DKFZP564O0823 gene is yet available in PubMed, the Gene Expression Omnibus (http://www.ncbi.nlm.nih.gov/geo/) comprises experimental microarray data with significantly altered expression of the human DKFZP564O0823 gene that point towards a role in inflammation and immune response. In a first study, primary human endothelial cells derived from different tissues were challenged with inflammatory and immune cytokines. The expression of DKFZP564O0823 was increased in primary colon endothelial cells upon exposure to TNFα as compared to exposure to IFNγ or IL-4 (Figure 6A). The latter two are primarily associated with T helper cell subsets, whereas TNFα is a pleiotropic cytokine with a critical function in both inflammatory and immunological responses. As these data on the colon endothelial cells form only a part of a large study, the publication on these experiments does not include the results on DKFZP564O0823, but rather focuses on well-known genes (Sana et al, Cytokine 2005; 29:256-269).

In a second study, gene expression was assessed in Jurkat CD4+ T cells following induction of the Nef protein from the simian immune deficiency virus (SIV) (Ndolo et al, Virology 2006; 353:374-387). The Nef protein is expressed early in SIV (and HIV) infections, and down-regulates major histocompatibility complex class I (MHC-I) molecules from the cell surface - thereby facilitating immune evasion. The microarray experiment reveals that - among many other genes that are well characterized - the expression of the DKFZP564O0823 gene is upregulated by SIV-Nef (Figure 6B).

These two latter studies in the literature are conceptually in line with our results that also point towards alterations in immune response in the colon of IBS patients compared to controls. The hypothesized biological link of the DKFZP564O0823 gene to IBS via an effect on immune response requires further functional characterization.

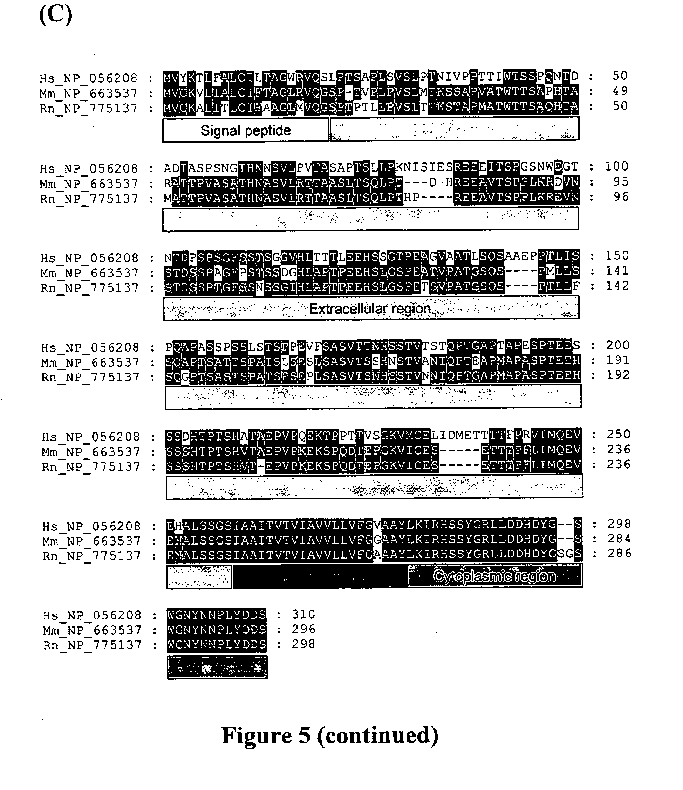

The gene is mainly expressed in colon and placenta, but is also found in many other tissues. Amino acid sequence alignment of the human, mouse, and rat homologues demonstrate a highly conserved sequence in the transmembrane and intracellular regions (95% and 94% amino acid sequence identity among the three species in these regions, respectively), but less homology in the extracellular region (51% AA sequence identity) (Figure 5C).
As no information on the functional role of IBS 1 is currently known, the consequence of the increased expression level in the colon of IBS patients is unclear. However, it is striking to note that two independent gene probe sets on the microarray identified this gene as the most significant in two independent analyses (SAM and mixed ANOVA) and that a relatively good discrimination of IBS from and health is possible solely based on these two probe sets (Figure 7, Figure 5B).

It is unclear whether the differentially expressed genes are the cause or rather the consequence of IBS. Because IBS is likely a complex multifactorial disorder, many genes may be involved, each with a relatively small contribution to the overall phenotype. Our results showing mainly subtle changes in expression of many genes in the colonic mucosa of IBS patients, support this concept. The altered expression of a specific gene was, in some cases, observed in only a subset of the IBS patients; this may reflect heterogeneity of the underlying molecular mechanisms with a common phenotype. Most of the genes with significant changes in expression are implicated in similar functional cellular processes involved in the host response to intraluminal antigen or bacterial invasion or their pro-inflammatory effects. Given this degree of mechanistic specificity in the identified genes, it is considered unlikely that they represent false positive associations picked up by chance using the two applied statistical methodologies.

### Potential for Molecular Diagnosis of IBS based on mucosal gene expression profiling

Because of its clinical relevance, the predictive power of a molecular diagnosis of IBS based on sigmoid colon expression profiles was subsequently assessed. The 61 subjects were therefore randomly divided into a training set and a test set. The training set (n=45) comprised 17 healthy subjects and 28 IBS patients (16 IBS-D, 12 IBS-C); the test set (n=16) comprised the remaining 8 healthy controls and 8 IBS patients (5 IBS-D, 3 IBS-C). Subsequently, two different and independent classification analysis methods were applied that that have been shown to perform well on datasets with many measurements and relatively few samples, as they are quite robust against overfitting. First, using Prediction Analysis for Microarrays (PAM) (Tibshirani et al., 2002) on the validation set, a 32 gene probe sets signature was obtained (Figure 8) with an average cross-validation misclassification rate of 22% (respectively 13/17 and 22/28 correctly classified for healthy and IBS subjects). Using this molecular signature on the independent samples in the test set, PAM correctly predicted the disease status of 75% of the participants, with an equally accurate prediction for both diseased and healthy persons. Overfitting did not seem to be an issue as the misclassification rates were similar for the training set (22%) and the test set (25%).

In order to further validate the molecular signature of 32 gene probe sets, reproducibility was determined. Thus, the molecular signature was highly reproducible between repeated samples in the same patient for the simultaneously collected samples (concordance 0.76 ± 0.05), as well as between two samples collected with an interval period of 2-3 months (concordance 0.67 ± 0.04). Within patient concordance significantly exceeded the overall concordance between participants (concordance -0.02 ±0.02; Mann-Whitney U test with unequal variances; respectively W = 3938, p<0.0001 and W = 7683, p<0.0001) (Figure 1B).

Finally, it was attempted to further reduce the number of probes in the molecular signature through the selection of gene probes that were identified in common by the PAM and a SAM analysis performed on the samples of the training set only. The resulting set of 16 probes, representing 11 different genes, was then used in an unsupervised classification method, hierarchical clustering, including all 61 subjects of the study (i.e. both the training and test set). This clustering method groups on the one hand the gene probes with a similar gene expression profile amongst the different subjects together, and on the other hand also groups the colonic samples with a similar expression profile over the 16 gene probes together into a cluster hierarchy, using average linkage and correlation as similarity measure (Figure 9A). At the first level of hierarchy of the gene probes (X-axis), a clear distinction was made between the gene probe sets that are up- versus down-regulated in IBS patients versus healthy controls. At the first level of hierarchy of the subjects (Y-axis), a subset of IBS patients is separated from all other subjects, which appears to be strongly determined by their low expression levels of F13A1, NCF4, M160, CSF1R, and/or FCGR2A. The second hierarchical clustering level further separates two groups, largely corresponding to the group of IBS patients and the healthy individuals. As might be expected for the samples of the training set that were used to select the gene probe sets for classification, a good separation was observed (respectively 14/17 and 25/28 correctly classified for the healthy subjects and the IBS patients). Overall, for the test set subjects, 11 out of 16 were correctly classified (69%), which is in line with the results of the above described PAM analysis. The positive predictive value (i.e., the probability that people with the molecular signature are indeed IBS patients: 6/8) was 75%, while the negative predictive value (i.e., the probability that people lacking the molecular signature are not IBS patients: 5/8) was 63%. Future studies will allow to more precisely determine the predictive value, the sensitivity and specificity, but it is already clear that these molecular signatures in the mucosal colon have the power to identify the majority of IBS patients. This finding demonstrates the possibility to define specific subgroups of IBS patients based on a common molecular signature, and hence paves the way towards more personalized medicine. The assessment of molecular signatures in mucosal colon biopsies provides a new complementary tool to help a clinician deciding on the diagnosis of IBS, and upon the most beneficial therapeutic strategy for an individual patient.
A potential influence of gender or drug treatment on the results should be considered. A graphical presentation of the distribution of gender and drug treatment over the cohort is included in Figure 9B, suggesting that any potential effect on the classification analysis by these potential confounders would have been only marginal. This was confirmed by an additional mixed ANOVA analysis using the set of signature genes. It was found that:
a. medication did not affect the expression level of these genes (all q>0.7 for testing medication effect)
b. there was no interaction between medication and disease indicating that the gene expression was not influenced by medication depending on disease status, that is health versus IBS (all q>0.1 for testing interaction between medication and disease status)
c. gender did not affect the expression level of these signature genes (all q>0.9 for testing medication effect) or the difference between health and IBS patients (all q>0.9 for testing interaction between gender and disease status).
**Confirmatory data for the changes in gene expression found by microarray analysis by independent technology, reverse-transcription quantitative polymerase chain reaction (RTQ-PCR)**

12 genes that were identified from the microarray data analysis were selected for confirmatory analysis by validated fluorogenic TaqMan gene expression assays-on-demand (Applied Biosystems). Normalisation of the TaqMan assay results was done relative to the control SART1 gene, because this gene was found earlier to be stable and is also moderately expressed in colon samples (Camilleri M et al, Gastroenterology 2007). Overall, the data show substantial concordance between Affymetrix microarray and TaqMan data when comparing the fold change in expression level between IBS patients and healthy subjects (Figure 10). With the exception of the FCGR2A gene, 11 out of the 12 genes analyzed showed the same trend of up- or down-regulated expression in IBS compared to healthy subjects. Significant differences (p<0.05) between IBS and healthy subjects were confirmed in 6 out of the 12 genes, and these represented the genes with the largest fold change values. This level of significance was not achieved for genes with a more subtle fold change difference. This can be explained, in part, by the fact that the TaqMan method only normalizes the gene expression data versus a single reference gene (SART1 in our assay), whereas the microarray method allows normalization of the expression level of each individual gene against all other genes on the microarray. This means that even relatively small gene expression variations of the SART1 gene will introduce noise (and variation) in the normalized expression level of the genes of interest using the RTQ-PCR technology. Thus, although the TaqMan technology has some advantages with regard to sensitivity compared to microarrays (i.e. genes with low expression can be analyzed using RTQ-PCR where microarray technology may fail), it is clear that relatively larger intra-group variations are often found for TaqMan assays as compared to the microarray analyses.

In summary, the data generated by TaqMan assays largely confirm the microarray data with regard to the fold change levels of the individual genes.

In conclusion, several differentially expressed genes are found in the colonic mucosa of IBS patients. Many of these genes are directed towards a change in host defense mechanisms. Proteins involved in host defense mechanisms, or those encoded by the differentially expressed genes, may represent potential targets for the development of novel drugs for IBS. The most significant gene with an elevated expression in IBS patients encodes for a poorly characterized membrane protein (DKFZP564O0823) of unclear function, for which the name IBS is proposed. Molecular signatures of gene expression in the colon, based on a limited set of genes, may be predictive of IBS disease status. These gene expression profiles are stable over several months, suggesting that the molecular signatures have potential to improve the diagnosis of IBS and monitor therapeutic effectiveness. At the very least, these biological differences in patients with IBS suggest that there are objective differences in the colon and that the disease does not represent exclusively a disorder of central nervous system perception.

## Claims

1. An *in vitro* method for detecting and/or monitoring Irritable Bowel Syndrome (IBS) in a subject, said method comprising:
(a) determining, in a biological sample of said subject, the level of gene transcription of at least the group consisting of:
- Irritable Bowel Syndrome 1 (IBS1) represented by SEQ ID NO:15,
- Caspase 1 dominant-negative inhibitor pseudo-ICE (COP1),
- Proteasome activator subunit 2 (PA28 beta) (PSME2),
- Coagulation factor XIII, A1 polypeptide (F13A1),
- Neutrophil cytosolic factor 4, 40kDa (NCF4),
- Colony stimulation factor-1 receptor (CSFR1),
- Scavenger receptor cysteine-rich type 1 protein (M160),
- potassium voltage-gated channel, delayed-rectifier, subfamily S, member 3 (KCNS3),
- V-set and immunoglobulin domain containing 2 (VSIG2),
- FC fragment of IgG, low affinity IIa, receptor (FCGR2A) and
- caspase 1, apoptosis related cysteine peptidase (CASP1),
(b) comparing the level of gene transcription with the level of gene transcription in a normal control sample; and
(c) determining the presence of IBS and/or its status based on the result from step (b).

2. The method according to claim 1 further comprising determining the level of gene transcription of:
- MUC 20 (Mucin 20),
- butyrophilin-like 8 (BTNL8),
- V-set and immunoglobulin domain containing, 4 (VSIG4),
- lysozyme (LYZ),
- activator of heat shock 90kDa protein ATPase homolog 2 (AHSA2),
- minichromosome maintenance deficient 5 (MCM5),
- C6orf111,
- Membrane-spanning 4-domains, subfamily A, member 4 (MS4A4A),
- Insulin-like growth factor 1 (IGF1),
- Helicase, lymphoid specific (HELLS),
- CD74 antigen,
- HRAS-like suppressor 2 (HRASLS2), and
- UDP-glucuronosyl transferase 2B15 (UGT2B15).

3. The method according to claim 1, wherein the level of gene transcription is determined using an array of probes selected from the probes listed in Table 1 or Table 2.

4. A diagnostic kit which consist of:
- a set of probes that specifically bind to the m RNA or nucleic acid molecules derived therefrom of IBS1 represented by SEQ ID NO:15, COP1, PSME2, F13A1, NCF4, CSFR1, M160, KCNS3 VSIG2, FCGR2A and CASP1.

5. Use of a set of probes that specifically bind to mRNA or nucleic acid molecules derived therefrom of the IBS-MSGs comprising IBS1 represented by SEQ ID NO:15, COP1, PSME2, F13A1, NCF4, CSFR1, M160, KCNS3 VSIG2, FCGR2A and CASP1, for the detection of IBS

6. The use of the set of probes of claim 5 in a method of claim 1.

## Patentansprüche

1. In-vitro-Verfahren zum Nachweisen und/oder Überwachen des Reizdarmsyndroms (Irritable Bowel Syndrome - IBS) in einem Subjekt, wobei das Verfahren umfasst:
(a) Bestimmen in einer biologischen Probe des Subjekts des Wertes einer Gentranskription zumindest der Gruppe bestehend aus:
- Reizdarmsyndrom 1 (IBS1), dargestellt durch SEQ ID NR:15,
- Caspase 1 dominant-negativer Inhibitor Pseudo-ICE (COP1),
- Proteasom Aktivator Subeinheit 2 (PA28 Beta) (PSME2),
- Gerinnungsfaktor XIII, A1 Polypeptid (F13A1),
- Neutrophilem zytosolischen Faktor 4, 40 kDa (NCF4),
- Koloniestimulierungsfaktor-1 Rezeptor (CSFR1),
- Scavenger Rezeptor Cystein-reiches Typ 1 Protein (M160),
- Spannungsgesteuertem Delayed-Rectifier Kaliumkanal, Unterfamilie S, Mitglied 3 (KCNS3)
- VSIG2 (V-Set and Immunoglobulin Domain Containing 2)
- FC-Fragment von IgG, geringe Affinität IIa, Rezeptor (FCGR2A) und
- Caspase 1, Apoptose verwandter Cystein Peptidase (CASP1)
(b) Vergleichen des Wertes der Gentranskription mit dem Wert der Gentranskription in einer normalen Kontrollprobe; und
(c) Bestimmen des Vorhandenseins von IBS und/oder seines Status auf der Basis des Ergebnisses von Schritt (b).

2. Verfahren nach Anspruch 1, des Weiteren umfassend das Bestimmen des Wertes einer Gentranskription von:
- MUC 20 (Mucin 20)
- Butyrophilin-artigem 8 (BTNL8)
- VSIG4 (V-Set and Immunoglobulin Domain Containing, 4),
- Lysozym (LYZ),
- Aktivator des Hitzeschock-90 kDa-Proteins ATPase Homolog 2 (AHSA2),
- MCM5 (Minichromosom Maintenance Deficient 5),
- C6orf111,
- Membran-überspannender 4-Domäne, Unterfamilie A, Mitglied 4 (MS4A4A),
- Insulinartigem Wachstumsfaktor 1 (IGF1),
- HELLS (Helicase, lymphoid specific),
- CD74 Antigen,
- HRAS-artiger Suppressor 2 (HRASLS2) und
- UDP-Glucuronosyl-Transferase 2B15 (UGT2B15)

3. Verfahren nach Anspruch 1, wobei der Wert der Gentranskription unter Verwendung einer Anordnung von Sonden bestimmt wird, die aus den Sonden ausgewählt sind, die in Tabelle 1 oder Tabelle 2 aufgelistet sind.

4. Diagnose-Kit, bestehend aus:
- einem Satz von Sonden, die spezifisch an die mRNA oder davon abgeleiteten Nukleinsäuremoleküle von IBS1, dargestellt durch SEQ ID NR:15, COP1, PSME2, F13A1, NCF4, CSFR1, M160, KCNS3, VSIG2, FCGR2A und CASP1 binden.

5. Verwendung eines Satzes von Sonden, die spezifisch an die mRNA oder davon abgeleiteten Nukleinsäuremoleküle der IBS-MSGs binden, umfassend IBS1, dargestellt durch SEQ ID NR:15, COP1, PSME2, F13A1, NCF4, CSFR1, M160, KCNS3, VSIG2, FCGR2A und CASP1, für den Nachweis von IBS.

6. Verwendung des Satzes von Sonden nach Anspruch 5 in einem Verfahren nach Anspruch 1.

## Revendications

1. Procédé *in vitro* pour détecter et/ou contrôler le Syndrome du Côlon Irritable (IBS) chez un sujet, ledit procédé comprenant :
(a) la détermination, dans un échantillon biologique dudit sujet, du niveau de transcription de gène au moins du groupe constitué par :
- le syndrome du côlon Irritable 1 (IBS1) représenté par SEQ ID n° 15,
- Caspase 1 inhibiteur dominant négatif pseudo-ICE (COP1),
- Sous-unité activatrice 2 de protéasome (PA28 bêta) (PSME2),
- Facteur de coagulation XIII, polypeptide A1 (F13A1),
- Facteur cytosolique neutrophile 4, 40kDa (NCF4),
- Récepteur de facteur 1 de stimulation de colonie (CSFR1),
- Protéine de type 1 riche en cystéine de récepteur de désactiveur (M160),
- Élément 3, sous-famille S, redresseur à retard, canal de potassium dépendant du potentiel (KCNS3),
- chaîne en V et domaine d'immunoglobuline contenant 2 (VSIG2),
- fragment FC d'IgG, faible affinité IIa, récepteur (FCGR2A) et
- caspase 1, apoptose liée à la peptidase à cystéine (CASP1),
(b) la comparaison du niveau de transcription de gène avec le niveau de transcription de gène dans un échantillon témoin normal ; et
(c) la détermination de la présence de IBS et/ou son état en se basant sur le résultat provenant de l'étape (b).

2. Procédé selon la revendication 1 comprenant en outre la détermination du niveau de transcription de gène de :
- MUC 20 (Mucine 20),
- semblable à butyrophiline 8 (BTNL8),
- chaîne en V et domaine d'immunoglobuline contenant 4 (VSIG4),
- Lysozyme (LYZ),
- activateur de protéine de choc thermique 90kDa ATPase homologue 2 (AHSA2),
- minichromosome maintenance déficient 5 (MCM5),
- C6orf111,
- Envergure de membrane à 4 domaines, sous-famille A, élément 4 (MS4A4A),
- facteur de croissance 1 semblable à l'Insuline (IGF1),
- Hélicase, lymphoïde spécifique (HELLS),
- antigène CD74,
- Suppresseur 2 semblable à HRAS (HRASLS2), et
- UDP-glucuronosyle transférase 2B15 (UGT2B15).

3. Procédé selon la revendication 1, dans lequel le niveau de transcription de gène est déterminé en utilisant une matrice de sondes sélectionnées à partir des sondes listées dans le Tableau 1 ou le Tableau 2.

4. Kit de diagnostic qui consiste en :
- un ensemble de sondes qui se lient de manière spécifique à l'ARNm ou aux molécules d'acide nucléique obtenues à partir de ce dernier d'IBS1 représenté par SEQ ID n° 15, COP1, PSME2, F13A1, NCF4, CSFR1, M160, KCNS3 VSIG2, FCGR2A et CASP1.

5. Utilisation d'un ensemble de sondes qui se lient de manière spécifique à l'ARNm ou à des molécules d'acide nucléique obtenues à partir de ce dernier des IBS-MSG comprenant IBS1 représenté par SEQ ID n° 15, COP1, PSME2, F13A1, NCF4, CSFR1, M160, KCNS3 VSIG2, FCGR2A et CASP1, pour la détection d'IBS.

6. Utilisation de l'ensemble de sondes selon la revendication 5 dans un procédé selon la revendication 1.


### SEQUENCE LISTING

<110> Janssen Pharmaceutica NV Mayo Clinic Rochester
<120> COMPOSITIONS AND METHODS FOR TREATING AND DIAGNOSING IRRITABLE BOWEL SYNDROME
<130>
<160> 16
<170> Patent In version 3.1
<210> 1
   <211> 456
   <212> DNA
   <213> Artificial Sequence
<220>
   <223> Affymetrix Probe ID No.:206011_at
<220>
   <221> misc_feature
   <222> (1) .. (456)
   <223> n represents any nucleotide
<400> 1
   aatgctctaaaatccaacactgtgtgagcgcccacatgatattcaaaggaaatgtttattga
   aacatttcaaattanaggtttttggattagggatgctaaaccagtaagtatacagctgaatt
   ccaatatacaaaaatatctgaaatttgaaatacttctggtagcatgcattttggataaggga
   taatcaagccatatacagaaaatactgaagtaatgcctttcttctggtcagtgcagagcacg
   ttgctcttctcccaaagtttttcatttagaccccttttgagattcatctgatattggcctag
   aagtggccgtaagactacaaaccctcacttttggatgtttctctccttcacctcagcagggt
   atatttaaacatagcatgtggtctttccttttaaaattgtgtatgttcccattggtggtata
   actttataacttgcatgtcttt
<210> 2
   <211> 311
   <212> DNA
   <213> Artificial Sequence
<220>
   <223> Affymetrix Probe ID No.:211367_s_at
<400> 2
   gagctgaggttgacatcacaggcatgacaatgctgctacaaaatctggggtacagcgtagat
   gtgaaaaaaaatctcactgcttcggacatgactacagagctggaggcatttgcacaccgccc
   agagcacaagacctctgacagcacgttcctggtgttcatgtctcatggtattcgggaaggca
   tttgtgggaagaaacactctgagcaagtcccagatatactacaactcaatgcaatctttaac
   atgttgaataccaagaactgcccaagtttgaaggacaaaccgaaggtgatcatcatccaggc
   c
<210> 3
   <211> 548
   <212> DNA
   <213> Artificial Sequence
<220>
   <223> Affymetrix Probe ID No.:211366_x_at
<400> 3
   gcacaagacctctgacagcacgttcctggtgttcatgtctcatggtattcgggaaggcattt
   gtgggaagaaacactctgagcaagtcccagatatactacaactcaatgcaatctttaacatg
   ttgaataccaagaactgcccaagtttgaaggacaaaccgaaggtgatcatcatccaggcctg
   ccgtggtgacagccctggtgtggtgtggtttaaagattcagtaggagtttctggaaacctat
   ctttaccaactacagaagagtttgaggatgatgctattaagaaagcccacatagagaaggat
   tttatcgctttctgctcttccacaccagataatgtttcttggagacatcccacaatgggctc
   tgtttttattggaagactcattgaacatatgcaagaatatgcctgttcctgtgatgtggagg
   aaattttccgcaaggttcgattttcatttgagcagccagatggtagagcgcagatgcccacc
   actgaaagagtgactttgacaagatgtttctacctcttcccaggacattaaa
<210> 4
   <211> 387
   <212> DNA
   <213> Artificial Sequence
<220>
   <223> Affymetrix Probe ID No.:209970_x_at
<400> 4
   cgaaggtgatcatcatccaggcctgccgtggtgacagccctggtgtggtgtggtttaaagat
   tcagtaggagtttctggaaacctatctttaccaactacagaagagtttgaggatgatgctat
   taagaaagcccacatagagaaggattttatcgctttctgctcttccacaccagataatgttt
   cttggagacatcccacaatgggctctgtttttattggaagactcattgaacatatgcaagaa
   tatgcctgttcctgtgatgtggaggaaattttccgcaaggttcgattttcatttgagcagcc
   agatggtagagcgcagatgcccaccactgaaagagtgactttgacaagatgtttctacctct
   tcccaggacattaaa
<210> 5
   <211> 210
   <212> DNA
   <213> Artificial Sequence
<220>
   <223> Affymetrix Probe ID No.:211368_s_at
<400> 5
   aatgtttcttggagacatcccacaatgggctctgtttttattggaagactcattgaacatat
   gcaagaatatgcctgttcctgtgatgtggaggaaattttccgcaaggttcgattttcatttg
   agcagccagatggtagagcgcagatgcccaccactgaaagagtgactttgacaagatgtttc
   tacctcttcccaggacattaaaat
<210> 6
   <211> 206
   <212> DNA
   <213> Artificial Sequence
<220>
   <223> Affymetrix Probe ID No.:1552703_s_at
<400> 6
   ttccatgggtgaaggtacaataaatggcttactggatgaattattacagacaagggtgctga
   accaggaagagatggagaaagtaaaacgtgaaaatgctacagttatggataagacccgagct
   ttgattgactccgttattccgaaaggggcacaggcatgccaaatttgcatcacatacatttg
   tgaagaagacagttacctgg
<210> 7
   <211> 55
   <212> DNA
   <213> Artificial sequence
<220>
   <223> Affymetrix Probe ID No.:1552701_a_at
<400> 7
   aggtccgatacctggaaattagcttagtacacaagactcccaattactattttct
<210> 8
   <211> 295
   <212> DNA
   <213> Artificial Sequence
<220>
   <223> Affymetrix Probe ID No.:201762_s_at
<400> 8
   caacacctgatccccaagattgaagatggaaatgattttggggtagcaatccaggagaaggt
   gctggagagggtgaatgccgtcaagaccaaagtggaagctttccagacaaccatttccaagt
   acttctcagaacgtggggatgctgtggccaaggcctccaaggagactcatgtaatggattac
   cgggccttggtgcatgagcgagatgaggcagcctatggggagctcagggccatggtgctgga
   cctgagggccttctatgctgagctttatcatatcatcagcagcaacc
<210> 9
   <211> 454
   <212> DNA
   <213> Artificial Sequence
<220>
   <223> Affymetrix Probe ID No.:203305_at
<400> 9
   gtccttcacatcaccattttgagacctcagcttggcactcaggtgctgaagggtaatatgga
   ctcagccttgcaaatagccagtgctagttctgacccaaccacagaggatgctgacatcattt
   gtattatgttccaaggctactacagagaaggctgcctgctatgtatttgcaaggctgattta
   tggtcagaatttccctctgatatgtctagggtgtgatttaggtcagtagactgtgattctta
   gcaaaaaatgaacagtgataagtatactgggggcaaaatcagaatggaatgctctggtctat
   ataaccacatttctgagcctttgagactgttcctgagccttcagcactaacctatgagggtg
   agctggtcccctctatatatacatcatacttaactttactaagtaatctcacagcatttgcc
   aagtctcccaatatccaatt
<210> 10
   <211> 497
   <212> DNA
   <213> Artificial Sequence
<220>
   <223> Affymetrix Probe ID No.:205147_x_at
<400> 10
   agcagaggctctatttgacttcactggaaacagcaaactggagctgaatttcaaagctggag
   atgtgatcttcctcctcagtcggatcaacaaagactggctggagggcactgtccggggagcc
   acgggcatcttccctctctccttcgtgaagatcctcaaagacttccctgaggaggacgaccc
   caccaactggctgcgttgctactactacgaagacaccatcagcaccatcaaggacatcgcgg
   tggaggaagatctcagcagcactcccctattgaaagacctgctggagctcacaaggcgggag
   ttccagagagaggacatagctctgaattaccgggacgctgagggggatctggttcggctgct
   gtcggatgaggacgtagcgctcatggtgcggcaggctcgtggcctcccctcccagaagcgcc
   tcttcccctggaagctgcacatcacgcagaaggacaactacagggtctacaacacgatgcca
   t
<210> 11
   <211> 440
   <212> DNA
   <213> Artificial Sequence
<220>
   <223> Affymetrix Probe ID No.:223655_at
<400> 11
   tctatgggactgtcacgccaaaccctggggacagagtgactgtggacacaaggaagatgctg
   gcgtgaggtgctctggacagtcgctgaaatcactgaatgcctcctcaggtcgtttagcactt
   attttatccagtatctttgggctccttctcccggttctgtttattctatttctcacgtggtg
   ccgagttcagaaacaaaaacatctgcccctcagagtttcaaccagaaggaggggttctctcg
   aggagaatttattccatgagatggagacctgcctcaagagagaggacccacatgggacaaga
   acctcagatgacacccccaaccatggttgtgaagatgctagcgacacatcgctgttgggagt
   tcttcctgcctctgaagccacaaaatgactttagacttccagggctcaccagatcaacctct
   aaatat
<210> 12
   <211> 482
   <212> DNA
   <213> Artificial Sequence
<220>
   <223> Affymetrix Probe ID No.:203104_at
<400> 12
   tgttggcctcgtgtttgctatgccaactagtagaaccttctttcctaatccccttatcttca
   tggaaatggactgactttatgcctatgaagtccccaggagctacactgatactgagaaaacc
   aggctctttggggctagacagactggcagagagtgagatctccctctctgagaggagcagca
   gatgctcacagaccacactcagctcaggccccttggagcaggatggctcctctaagaatctc
   acaggacctcttagtctctgccctatacgccgccttcactccacagcctcacccctcccacc
   cccatactggtactgctgtaatgagccaagtggcagctaaaagttgggggtgttctgcccag
   tcccgtcattctgggctagaaggcaggggaccttggcattggctggccacaccaagcaggaa
   gcacaaactcccccaagctgactcatcctaactaacagtcacgccgtg
<210> 13
   <211> 515
   <212> DNA
   <213> Artificial Sequence
<220>
   <223> Affymetrix Probe ID No.:203561_at
<400> 13
   tgctgggatgaccagcatcagccccaatgtccagcctctttaacatcttctttcctatgccc
   tctctgtggatccctactgctggtttctgccttctccatgctgagaacaaaatcacctattc
   actgcttatgcagtcggaagctccagaagaacaaagagcccaattaccagaaccacattaag
   tctccattgttttgccttgggatttgagaagagaattagagaggtgaggatctggtatttcc
   tggactaaattccccttggggaagacgaagggatgctgcagttccaaaagagaaggactctt
   ccagagtcatctacctgagtcccaaagctccctgtcctgaaagccacagacaatatggtccc
   aaatgactgactgcaccttctgtgcctcagccgttcttgacatcaagaatcttctgttccac
   atccacacagccaatacaattagtcaaaccactgttattaacagatgtagcaacatgaaaga
   cgctatgttacaggttaca
<210> 14
   <211> 477
   <212> DNA
   <213> Artificial Sequence
<220>
   <223> Affymetrix Probe ID No.:205968_at
<400> 14
   attgtggtgagcgatcctgactccacagatgcttcaagcattgaagacaatgaggacatttg
   taacaccacctccttggagaattgcacagcaaaatgagcgggggtgtttgtgcctgtttctc
   ttatcctttcccaacattaggttaacacagctttataaacctcagtgggttcgttaaaatca
   tttaattctcagggtgtacctttccagccatagttggacattcattgctgaattctgaaatg
   atagaattgtctttatttttctctgtgaggtcaattaaatgccttgttctgaaatttatttt
   ttacaagagagagttgtgatatagtttggaatataagataaatggtattgggtggggtttgt
   ggctacagcttatgcatcattctgtgtttgtcatttactcacattgagctaactttaaatta
   ctgacaagtagaatcaaaggtgcagctgactgagacgacatgc
<210> 15
   <211> 512
   <212> DNA
   <213> Artificial Sequence
<220>
   <223> Affymetrix Probe ID No.:204687_at
<400> 15
   aatctctatttatctggttgtttctgacaggatgctgcctgcttggctctacaagctggaaa
   gcagcttcttagctgcctaattaatgaaagatgaaaataggaagtgccctggagggggccag
   caggtcacggggcagaatctctcaggttgctgtgggatctcagtgtgcccctacctgttctc
   ccctccaggccacctgtctctgtaaaggatgtctgctctgttcaaaaggcagctgggatccc
   agcccacaagtgatcagcagagttgcatttccaaagaaaaaggctatgagatgagctgagtt
   atagagagaaagggagaggcatgtacggtgtggggaagtggaagggaagctggcgggggaga
   aggaggctaacctgcactgagtacttcattaggacaagtgagaatcagctattgataatggc
   cagagatatccacagcttggaggagcccagagaccgtttgctttatacccacacagcaactg
   gtccactgctttactg
<210> 16
   <211> 228
   <212> DNA
   <213> Artificial Sequence
<220>
   <223> Affymetrix Probe ID No.:229369_at
<220>
   <221> misc_feature
   <222> (1)..(228)
   <223> n represents any nucleotide
<400> 16
   ggggtggcgcaaggagggaggaaagggcttgagttaaaagcgggtgcctgcaaccctcaaac
   tccgacatcattcagtgtgtttaggggcaggaggtgttgttcagccgtggaatttgctggtg
   gcagcagtgtaacctgtgtatttgagggtacaggcaancggtacagggtggagtggctggtc
   cacaagctgtggcagggaagctgtttgcaggactgccctgcc